# EUROPEAN PATENT SPECIFICATION

(11) **EP 3 773 844 B1**
(45) Date of publication and mention of the grant of the patent: **24.04.2024**
(21) Application number: 19775830.3
(22) Date of filing: 28.03.2019
(51) Int. Cl.: A61M 16/06

(54) **PATIENT INTERFACE**
PATIENTENSCHNITTSTELLE
INTERFACE PATIENT

(30) Priority: 28.03.2018 US 201862649376 P
(43) Date of publication of application: 17.02.2021
(73) Proprietor: ResMed Pty Ltd, Bella Vista, NSW 2153 (AU)
(72) Inventor: EVES, Matthew, New South Wales 2153 (AU); GOLDSPINK, Lachlan Richard, New South Wales 2153 (AU)
(74) Representative: Vossius & Partner Patentanwälte Rechtsanwälte mbB
(86) International application number: PCT/AU2019/050279
(87) International publication number: WO 2019/183681

(56) References cited:
- WO-A1-2008/011682
- WO-A1-2013/066195
- WO-A1-2015/020535
- WO-A1-2015/020535
- WO-A1-2015/041545
- WO-A1-2017/037638
- WO-A1-2017/185140
- US-A1- 2016 082 214
- US-A1- 2016 082 214
- US-A1- 2017 246 411
- US-B2- 8 402 971

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

US 2016/082214 A1 relates to a patient interface to provide breathable gas to a patient. The patient interface comprises a plenum chamber assembly, comprising a nasal plenum chamber at least partly defining an upper gas chamber; an oral plenum chamber at least partly defining a lower gas chamber, and a decoupling structure at least partly connecting the nasal plenum chamber and the oral plenum chamber and at least party defining a flow path, a top plate including at least one connection feature configured to releasably retain a first portion of a positioning and stabilising structure, and a faceplate configured to releasably retain a second portion of a positioning and stabilising structure.

WO 2017/037638 A1 relates to an interface for positive pressure respiratory therapy. The interface includes a mask assembly having a mask seal and a mask shell. The mask assembly is positioned lower than and exposes a bridge of the user's nose. The mask seal includes first and second portions on respective first and second sides of a nasal region that contact opposing sides of the user's nose. In some configurations, the first and second portions each include supports that help maintain a shape of the mask seal. A pair of covers can be supported relative to the mask assembly and adjacent a respective one of the first and second portions of the mask seal. The covers limit expansion of the first and second portions of the mask seal in response to pressurized air within the mask seal. The supports of the first and second portions can transfer load from the mask seal to the covers. An interface system can include multiple size mask seals, which can be designed with consideration of ethnic variations in nose size and/or shape.

US 8 402 971 B2 relates to a mouth cushion for a mask system including a side wall, an undercushion extending away from the side wall, and a membrane provided to substantially surround the undercushion and adapted to form a continuous seal around an exterior of a patient's mouth in use. The side wall includes spaced-apart prong support structures that provide annular recesses adapted to support respective nasal prongs, and at least a portion of the undercushion includes a question-mark or sickleshape configuration when in cross-section.

### 1.2.2 Therapy

Various therapies, such as Continuous Positive Airway Pressure (CPAP) therapy, Non-invasive ventilation (NIV) and Invasive ventilation (IV) have been used to treat one or more of the above respiratory disorders.

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 1.2.3 Treatment Systems

These therapies may be provided by a treatment system or device. Such systems and devices may also be used to screen, diagnose, or monitor a condition without treating it.

A treatment system may comprise a Respiratory Pressure Therapy Device (RPT device), an air circuit, a humidifier, a patient interface, and data management.

Another form of treatment system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 1.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressurised. Examples of RPT devices include a CPAP device and a ventilator.

### 1.2.3.3 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air.

### 1.2.3.4 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.3.5 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient's teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient's mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC^{™} MRD are designed to retain the mandible in a forward position. This device also reduces or minimises dental and temporo-mandibular joint (TMJ) side effects. Thus, it is configured to minimises or prevent any movement of one or more of the teeth.

### 1.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH₂O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{™} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26 (3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 1.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 2 BRIEF SUMMARY OF THE TECHNOLOGY

The invention is defined in independent claim 1. Preferred embodiments are matter of the appended dependent claims. Subject-matter referred as embodiments, aspects and/or disclosures which are not covered by the claims are not part of the invention.

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

According to a first aspect of the present technology, there is provided a plenum chamber for a patient interface, the plenum chamber pressurisable to a therapeutic pressure of at least 6 cmH₂O above ambient air pressure, the plenum chamber having an oral portion and a nasal portion, the plenum chamber comprising: a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the one or more holes, the seal-forming structure constructed and arranged to maintain said therapeutic pressure in the plenum chamber throughout the patient's respiratory cycle in use; a shell having one or more plenum chamber inlet ports sized and structured to receive a flow of air at the therapeutic pressure for breathing by a patient the shell supporting the seal-forming structure, wherein the shell is provided to the oral portion of the plenum chamber, and substantially the entire nasal portion is formed by the seal-forming structure; and wherein the seal-forming structure comprises lateral support portions located on laterally-spaced, at least partially anterior-facing sides of the nasal portion, the lateral support portions having a higher resistance to deformation in use in comparison to one or more adjacent portions of the seal-forming structure.

According to another aspect of the present technology, there is provided a plenum chamber for a patient interface, the plenum chamber pressurisable to a therapeutic pressure of at least 6 cmH₂O above ambient air pressure, the plenum chamber having an oral portion and a nasal portion, the plenum chamber comprising: a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the one or more holes, the seal-forming structure constructed and arranged to maintain said therapeutic pressure in the plenum chamber throughout the patient's respiratory cycle in use; wherein substantially the entire nasal portion is formed by the seal-forming structure; and wherein the seal-forming structure comprises lateral support portions located on laterally-spaced, at least partially anterior-facing sides of the nasal portion, the lateral support portions having a higher resistance to deformation in use in comparison to one or more adjacent portions of the seal-forming structure.

In examples: the lateral support portions are portions of the seal-forming structure having a relatively greater stiffness in comparison to one or more adjacent portions of the seal-forming structure; the lateral support portions are portions of the seal-forming structure supported by a support structure; the support structure is a portion of a frame or a clip-on support; the lateral support portions comprise portions of the seal-forming structure having relatively greater thickness in comparison to one or more adjacent portions of the seal-forming structure; the lateral support portions are provided substantially directly superior to portions the shell; the lateral support portions each have a substantially flat inferior boundary; the flat inferior boundary is provided adjacent a superior edge of the shell; the lateral support portions each have a curved superior boundary. Curvature of the superior boundary substantially matches or follows curvature of a superior periphery of the seal-forming structure; the lateral support portions are substantially fin-shaped.

In examples: the seal-forming structure comprises a central portion configured to form a seal to an inferior periphery of the patent's nose surrounding the patient's nares and to the patient's lip superior in use, the central portion having a lesser thickness than the lateral support portions; the seal-forming structure comprises intermediate portions provided between the central portion and the lateral support portions, the seal-forming structure having a greater thickness in the intermediate portions than in the central portion; the seal-forming structure comprises a lesser thickness in the intermediate portions than in the lateral support portions; the seal-forming structure comprises lateral-facing posterior portions provided between respective intermediate portions and lateral support portions on each side of the nasal portion, the seal-forming structure having a greater thickness in the posterior lateral facing portions than in the intermediate portions; at least part of each intermediate portion is provided superior and anterior to a respective lateral support portion; at least part of each lateral-facing posterior portion is provided superior and posterior to a respective lateral support portion.

In examples: the seal-forming structure comprises, on each lateral side of the nasal portion, a junction between a respective lateral support portion, intermediate portion and lateral-facing posterior portion, the junction being located proximate a superior-most point of the lateral support portion; the junction is located anterior to the superior-most point of the lateral support portion.

In examples: the central portion of the seal-forming structure comprises an anterior-facing central portion and a superior-facing central portion; the superior-facing central portion and anterior-facing central portion being connected to each other by a central saddle region of the nasal portion of the seal-forming structure; the superior facing central portion has a positive curvature in a lateral direction, sides of the superior-facing central portion facing in a partially medial direction.

In examples: the intermediate portions each comprise a superior-facing central portion and an anterior-facing central portion; the superior-facing central portion and anterior-facing central portion are connected to each other over a superior periphery of the seal-forming structure; the nasal portion comprises a lip superior portion configured to seal against the lip superior of the patient in use; the lip superior portion has a stiffness similar to a stiffness of the central portion; the lip superior portion has a wall thickness substantially equal to that of the central portion; the lip superior portion comprises a wall thickness lesser than a wall thickness of the intermediate portions.

In examples: the nasal portion comprises posterior corners configured to lie on the patients face proximate nasolabial creases on the patient's face; the posterior corners are configured to contact the patient's face on respective lateral sides of the lip superior; the posterior corners are configured to contact the patient's face proximate an area lateral and inferior to the nasal ala; the posterior corners are configured to fit between the patient's nasal ala and the patient's nasolabial sulci.

In examples: the posterior corners have a stiffness greater than a stiffness of the central portion of the nasal portion of the seal-forming structure; the posterior corners have a wall thickness greater than that of the intermediate portions of the nasal portion; the posterior corners have a wall thickness lesser than the lateral-facing posterior portions of the nasal portion; the posterior corners are substantially domeshaped; the transition between the posterior corners and the lip superior portion is configured to be located inferior to the patient's nasal ala in use.

In examples: the shell comprises posteriorly protruding portions reinforcing the seal-forming structure at a base of the nasal portion; the posteriorly protruding portions are provided at superior lateral corners of the shell; the posterior pointing portions are provided inferior to the lateral support portions; the posterior pointing portions have a flat superior edge; a flat inferior boundary of each lateral support portions is provided adjacent each posterior pointing portion.

In examples: the oral portion comprises a lip inferior portion configured to form a seal to the lip inferior of the patient, the lip inferior portion having a wall thickness substantially equal to that of the lip superior portion; the oral portion comprises an oral hole peripheral portion, the oral hole peripheral portion comprising a wall thickness substantially equal to lip superior portion; the oral hole peripheral portion is contiguous with either or both of the lip superior portion and the lip inferior portion; the oral portion comprises posterior-facing lateral portions provided to either lateral side of the oral hole peripheral portion configured to seal against the patient's cheeks in use; the posterior-facing lateral portions comprise a wall thickness greater than that of the oral hole peripheral portion; the posterior-facing lateral portions curve away from contact with the patient's face; the lip inferior portion is approximately half the width of the oral opening; the transitions between the lip inferior portion and the posterior-facing lateral portions on either lateral side of the lip inferior portion are configured to lie at or proximate labiomandibular creases of the patient's face; the lip inferior portion is wider at the periphery of the oral hole than at the lower periphery of the seal-forming structure.

In examples: the oral portion comprises lateral portions at the lateral periphery of the seal forming structure; the lateral portions comprise a wall thickness greater than that of the posterior-facing lateral portions; the oral portion comprises anterior-facing lateral portions on the anterior side of the seal-forming structure; the anterior-facing lateral portions comprise a greater wall thickness than that of the lateral portions; the oral portion comprises anterior support portions on the anterior side of the seal-forming structure; the anterior support portions comprise a greater wall thickness than that of the anterior-facing lateral portions; the oral portion comprises two anterior support portions proximate superior lateral corners of the shell; the oral portion comprises two anterior support portions proximate inferior lateral corners of the shell.

In examples: the plenum chamber comprises a single plenum chamber inlet port; the single plenum chamber inlet port is provided centrally in the shell; the plenum chamber inlet port is configured to connect to a frame; the plenum chamber inlet port is substantially circular.

According to another aspect of the present technology there is provided a plenum chamber for a patient interface, the plenum chamber pressurisable to a therapeutic pressure of at least 6 cmH₂O above ambient air pressure, the plenum chamber having an oral portion and a nasal portion, the plenum chamber comprising: a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the holes, the seal-forming structure constructed and arranged to maintain said therapeutic pressure in the plenum chamber throughout the patient's respiratory cycle in use; a shell configured to support the seal-forming structure, the shell having one or more plenum chamber inlet ports sized and structured to receive a flow of air at the therapeutic pressure for breathing by a patient; wherein the shell comprises lateral support portions protruding in a superior direction into the nasal portion of the patient interface on laterally-spaced, at least partially anterior-facing sides of the nasal portion.

In examples: the lateral support portions each have a curved superior boundary; curvature of the superior boundary substantially matches or follows curvature of a superior periphery of the seal-forming structure; the lateral support portions are substantially fin-shaped.

In examples: the plenum chamber comprises two plenum chamber inlet ports; the plenum chamber inlet ports are provided to lateral sides of the shell; the plenum chamber inlet ports are configured to connect to conduits; the plenum chamber inlet ports are approximately elliptical; the superior periphery of each of the two plenum chamber inlet ports is formed by a respective one of the lateral support portions.

According to another aspect of the present technology there is provided a plenum chamber for a patient interface, the plenum chamber being pressurisable to a therapeutic pressure of at least 6 cmH₂O above ambient air pressure, the plenum chamber comprising: one or more walls at least partially enclosing a volume of space; a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the one or more holes, the seal-forming structure constructed and arranged to maintain said therapeutic pressure in the plenum chamber throughout the patient's respiratory cycle in use, one or more plenum chamber inlet ports sized and structured to receive a flow of air at the therapeutic pressure for breathing by a patient; wherein the seal-forming structure comprises a central portion configured to seal to an inferior periphery of the patient's nose surrounding the patient's nares and to the patient's lip superior in use, and intermediate portions configured to be located against or proximate the ala of the patient's nose in use, the intermediate portions having a greater stiffness than the central portion.

In examples: the one or more walls may comprise some or all of the seal-forming structure; the one or more walls may be separate to the seal-forming structure; the intermediate portions comprise a pair of exterior walls of the seal-forming structure facing in partially medial and partially superior directions; the intermediate portions are configured to resist creasing; the intermediate portions are configured to limit creases from forming leak paths past the inferior periphery of the patient's nose to ambient.

According to another aspect of the present technology there is provided a plenum chamber for a patient interface, the plenum chamber being pressurisable to a therapeutic pressure of at least 6 cmH₂O above ambient air pressure, the plenum chamber comprising: one or more walls at least partially enclosing a volume of space; a seal-forming structure constructed and arranged to form a seal with a region of the patient's face surrounding an entrance to the patient's airways, said seal-forming structure having at least one hole therein such that the flow of air at said therapeutic pressure is delivered to an entrance to the patient's nares and to the patient's mouth via the at least one hole, the seal-forming structure constructed and arranged to maintain said therapeutic pressure in the plenum chamber throughout the patient's respiratory cycle in use; one or more plenum chamber inlet ports sized and structured to receive a flow of air at the therapeutic pressure for breathing by a patient; wherein the seal-forming structure comprises an oral hole peripheral portion surrounding at least a majority of an oral hole configured to surround the patient's mouth in use, and lateral peripheral support portions provided at opposite lateral sides of the oral hole, the lateral peripheral support portions having a stiffness greater than a stiffness of the oral hole peripheral portion.

In examples: the lateral peripheral support portions comprise a wall thickness greater than a wall thickness of the oral hole peripheral portion; the seal-forming structure comprises posterior-facing lateral portions surrounding a majority of the oral hole peripheral portion, the posterior-facing lateral portions having a thickness greater than the oral hole peripheral portion; the posterior-facing lateral portions form the lateral peripheral support portions. The posterior-facing lateral portions extend medially towards the lateral-most edges of the oral hole to form the lateral peripheral support portions; the lateral support portions provide resistance to buckling in the seal forming structure.

According to another aspect of the present technology there is provided a plenum chamber for a patient interface, the plenum chamber being pressurisable to a therapeutic pressure of at least 6 cmH₂O above ambient air pressure, the plenum chamber comprising: one or more walls at least partially enclosing a volume of space; one or more plenum chamber inlet ports sized and structured to receive a flow of air at the therapeutic pressure for breathing by a patient; a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the one or more holes, the seal-forming structure constructed and arranged to maintain said therapeutic pressure in the plenum chamber throughout the patient's respiratory cycle in use; wherein the seal-forming structure comprises a nasal portion configured to form a seal to an inferior periphery of the patient's nose in use, the nasal portion having a central portion configured to be located inferior to the patient's pronasale in use and intermediate portions configured to be located proximate to the patient's ala in use, the seal-forming structure having a greater stiffness in the intermediate portions than in the central portion.

In examples: the intermediate portions of the seal-forming structure comprise a greater wall thickness than the central portion; the intermediate portions may each comprise a medially-facing wall configured to be lie at or proximate the nasal ala of the patient in use; the central portion of the seal-forming structure comprises an anterior-facing central portion and a superior-facing central portion; the superior-facing central portion and anterior-facing central portion being connected to each other by a central saddle region of the nasal portion of the seal-forming structure; the superior facing central portion comprises a positive curvature in a lateral direction, sides of the superior-facing central portion facing in a partially medial direction; the intermediate portions each comprise a superior-facing central portion an anterior-facing central portion; the superior-facing central portion and anterior-facing central portion are connected to each other overall a superior periphery of the seal-forming structure; the central saddle portion comprises an equal wall thickness to the central portion; the anterior-facing central portion comprises a lesser wall thickness than the anterior-facing intermediate portions; when a downwards force is exerted on the central portion from the patient's nose, the sides of the nasal portion are drawn inwards towards the patient's nasal ala; the intermediate portions are drawn inwards to lie at or proximate the patient's ala; the nasal portion comprises a lip superior portion configured to seal against the lip superior of the patient in use; the lip superior portion comprises a stiffness similar to a stiffness of the central portion; the lip superior portion comprises a wall thickness substantially the equal to a wall thickness of the central portion; the lip superior portion comprises a wall thickness lesser than a wall thickness of the intermediate portions.

According to another aspect of the present technology there is provided a plenum chamber for a patient interface, the plenum chamber being pressurisable to a therapeutic pressure of at least 6 cmH₂O above ambient air pressure, the plenum chamber having comprising: one or more walls at least partially enclosing a volume of space; one or more plenum chamber inlet ports sized and structured to receive a flow of air at the therapeutic pressure for breathing by a patient; a seal-forming structure constructed and arranged to form a seal with a region of the patient's face surrounding an entrance to the patient's airways, said seal-forming structure having a plurality of holes therein such that the flow of air at said therapeutic pressure is delivered to an entrance to the patient's nares and to the patient's mouth via the plurality of holes, the seal-forming structure constructed and arranged to maintain said therapeutic pressure in the plenum chamber throughout the patient's respiratory cycle in use; wherein the plenum chamber comprises a nasal portion and the seal-forming structure in the nasal portion is configured to seal to an inferior periphery of the patient's nose, the seal-forming structure defining a pair of nasal holes configured in use to deliver the flow of air to the patient's nasal airways, the seal-forming structure comprising a bridge portion between the pair of nasal holes, the bridge portion being provided centrally between a central portion and a lip superior portion of the seal-forming structure in the nasal portion, the bridge portion being slack to allow movement of the central portion away from the posterior region.

In examples: the bridge portion comprises a curved portion configured to straighten when the central portion moves away from the lip superior portion; the curved portion comprises a single curve; the curved portion comprises two curves; the curved portion is approximately S-shaped when viewed from a lateral direction; the curved portion comprises a sawtooth shape; the curved portion comprises one or more folds; the bridge portion hangs inferior to the central portion and is able to straighten while the central portion moves away from the lip superior portion; the bridge portion enables the central portion to move to receive the patient's nose when the patient dons the patient interface; the bridge portion enables the central portion to move in an anterior direction to receive the pronasale of the patient when the patient dons the patient interface.

According to another aspect of the present technology there is provided a plenum chamber for a patient interface, the plenum chamber pressurisable to a therapeutic pressure of at least 6 cmH₂O above ambient air pressure, the plenum chamber comprising: one or more plenum chamber inlet ports sized and structured to receive a flow of air at the therapeutic pressure for breathing by a patient; a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the one or more holes, the seal-forming structure constructed and arranged to maintain said therapeutic pressure in the plenum chamber throughout the patient's respiratory cycle in use; wherein the seal-forming structure comprises a nasal portion configured to form a seal to the patient's face at or proximate the patient nose, and an oral portion configured to form a seal to the patient's face around the patient's mouth, the seal-forming structure having a first surface finish in the nasal portion and a second surface finish in the oral portion different from the first surface finish.

In examples: the first surface finish and the second surface finish differ in such a way that a coefficient of friction between the seal-forming structure and the patient's face is greater in the oral portion than in the nasal portion; the first surface finish is configured to provide the nasal portion with a smooth feel on the patient's face (e.g. for comfort); the second surface finish is configured to provide the oral portion with grippy contact on the patient's face (e.g. for a more robust seal); the first surface finish may be a frosted surface finish; the second surface finish may be a polished surface finish; a boundary between the first surface finish and the second surface finish may be located on a part of the seal-forming structure in contact with the patient's checks in use; the nasal portion may comprise a lip superior portion having the first surface finish; non-patient contacting surfaces of the nasal portion may comprise a surface finish other than the first surface finish; the non-patient contacting surfaces of the nasal portion may comprise the second surface finish.

According to another aspect of the present technology, there is provided a patient interface comprising: a plenum chamber according to an aspect of the present technology described above; a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use; and a vent structure to allow a continuous flow of gases exhaled by the patient from an interior of the plenum chamber to ambient, said vent structure being sized and shaped to maintain the therapeutic pressure in the plenum chamber in use; wherein the patient interface is configured to allow the patient to breath from ambient through their mouth in the absence of a flow of pressurised air through the plenum chamber inlet port.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:

### 3.1 TREATMENT SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

Fig. 2E is a further side view of a head. The approximate locations of the Frankfort horizontal and nasolabial angle are indicated. The coronal plane is also indicated.

Fig. 2F shows a base view of a nose with several features identified including naso-labial sulcus, lip inferior, upper Vermilion, naris, subnasale, columella, pronasale, the major axis of a naris and the midsagittal plane.

Fig. 2G shows a side view of the superficial features of a nose.

Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.

Fig. 2I shows a medial dissection of a nose, approximately several millimeters from the midsagittal plane, amongst other things showing the septum cartilage and medial crus of greater alar cartilage.

Fig. 2J shows a front view of the bones of a skull including the frontal, nasal and zygomatic bones. Nasal concha are indicated, as are the maxilla, and mandible.

Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

Fig. 2L shows an anterolateral view of a nose.

### 3.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows a cushion for a mask that includes two pillows. An exterior surface of the cushion is indicated. An edge of the surface is indicated. Dome and saddle regions are indicated.

Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

Fig. 3I shows the surface of a structure, with a one dimensional hole in the surface. The illustrated plane curve forms the boundary of a one dimensional hole.

Fig. 3J shows a cross-section through the structure of Fig.3I. The illustrated surface bounds a two dimensional hole in the structure of Fig. 3I.

Fig. 3K shows a perspective view of the structure of Fig. 31, including the two dimensional hole and the one dimensional hole. Also shown is the surface that bounds a two dimensional hole in the structure of Fig. 3I.

Fig. 3L shows a mask having an inflatable bladder as a cushion.

Fig. 3M shows a cross-section through the mask of Fig. 3L, and shows the interior surface of the bladder. The interior surface bounds the two dimensional hole in the mask.

Fig. 3N shows a further cross-section through the mask of Fig. 3L. The interior surface is also indicated.

Fig. 3O illustrates a left-hand rule.

Fig. 3P illustrates a right-hand rule.

Fig. 3Q shows a left ear, including the left ear helix.

Fig. 3R shows a right ear, including the right ear helix.

Fig. 3S shows a right-hand helix.

Fig. 3T shows a view of a mask, including the sign of the torsion of the space curve defined by the edge of the sealing membrane in different regions of the mask.

Fig. 3U shows a view of a plenum chamber 3200 showing a sagittal plane and a mid-contact plane.

Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

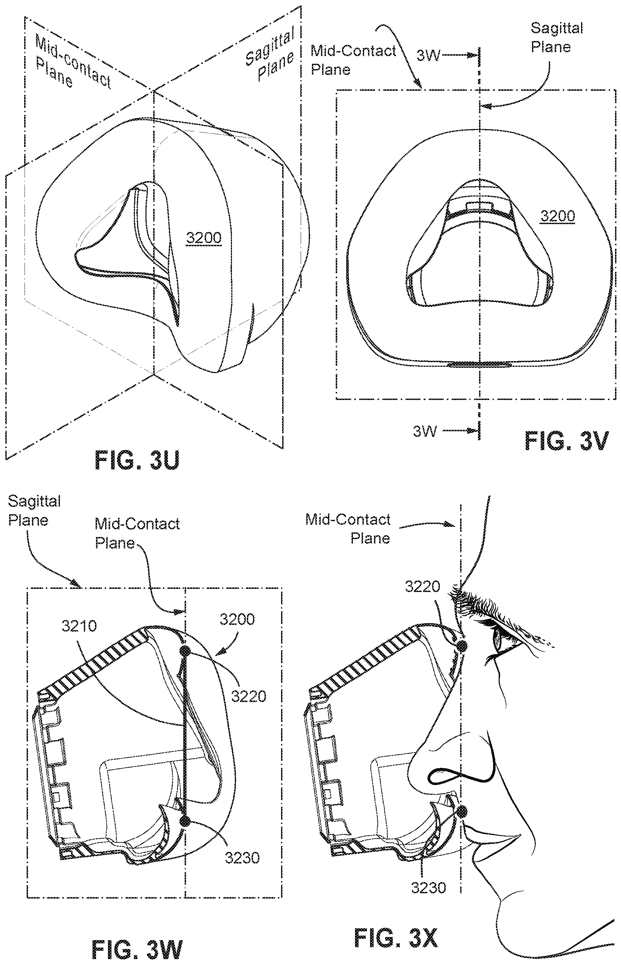

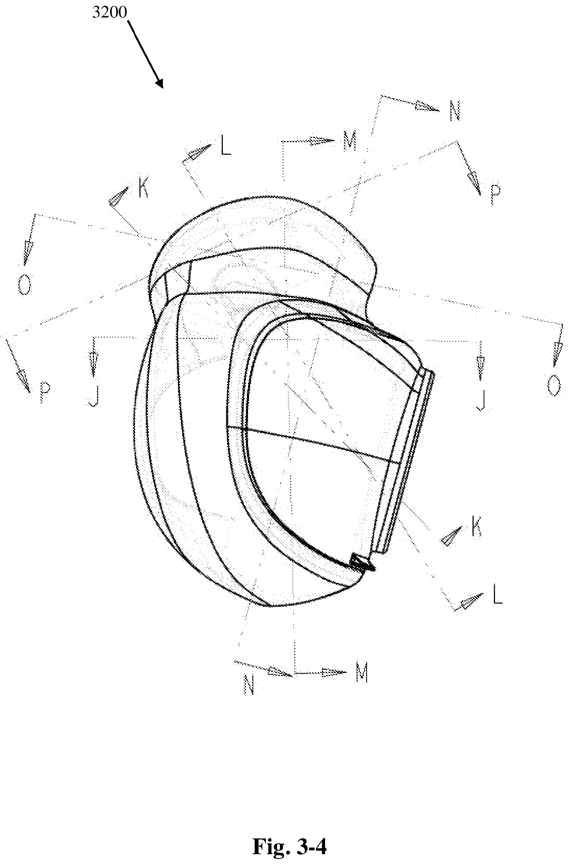

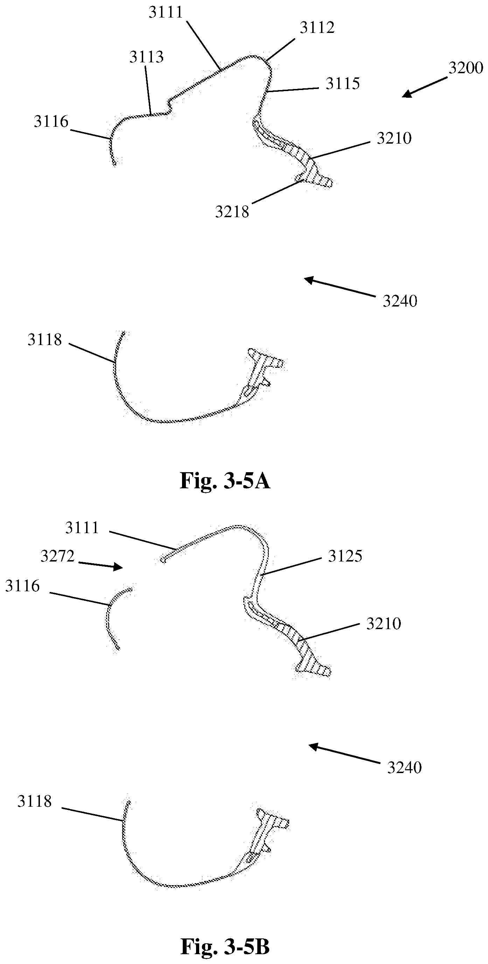

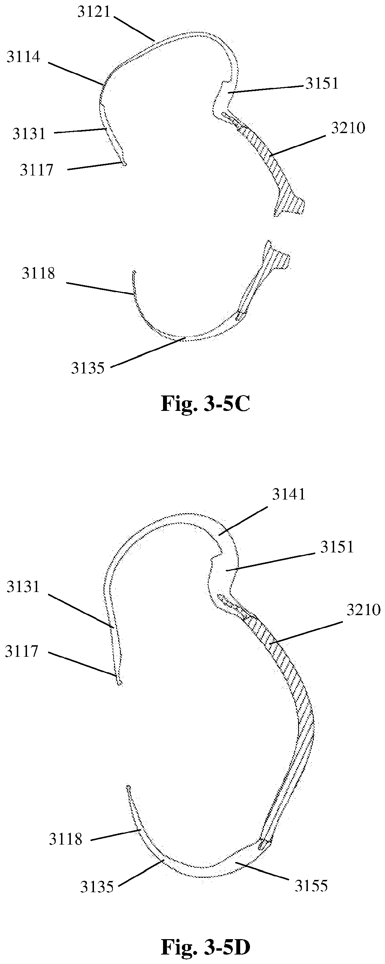

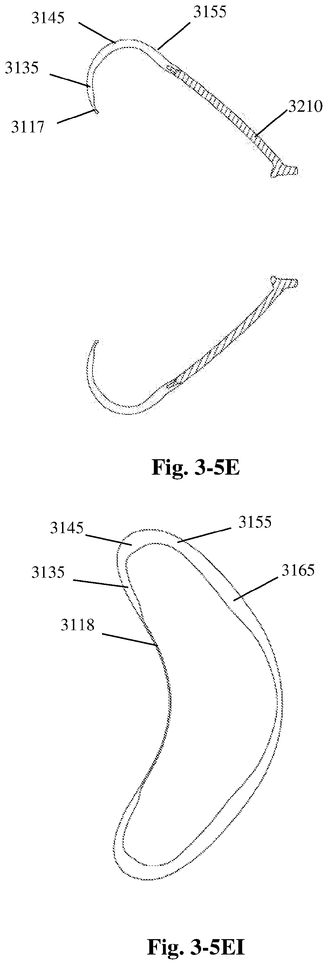

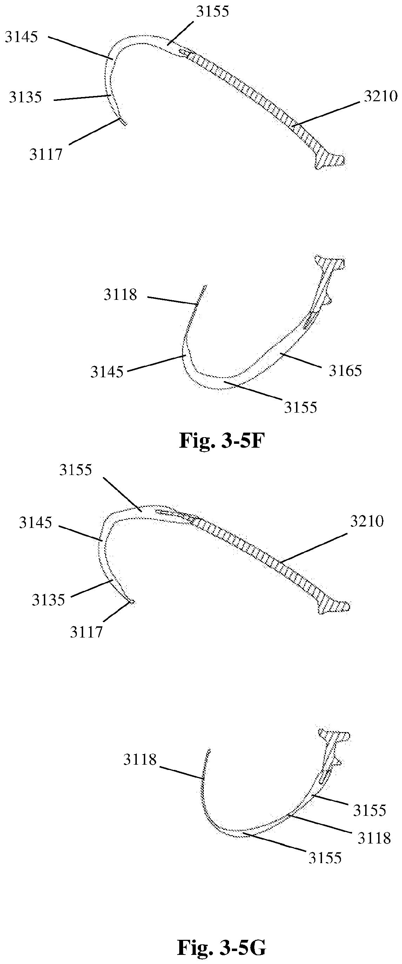

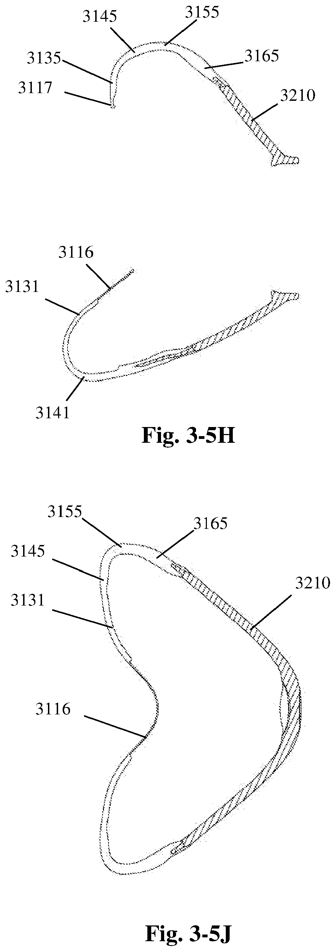

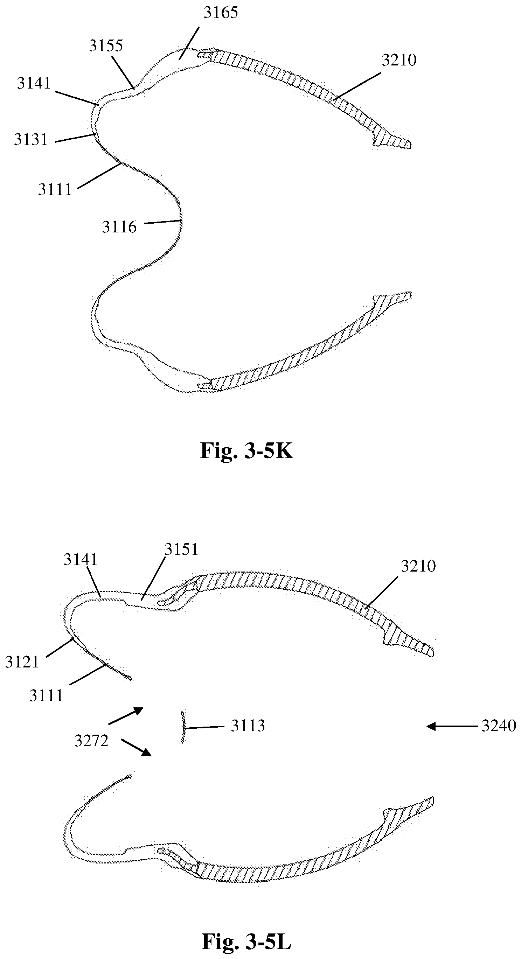

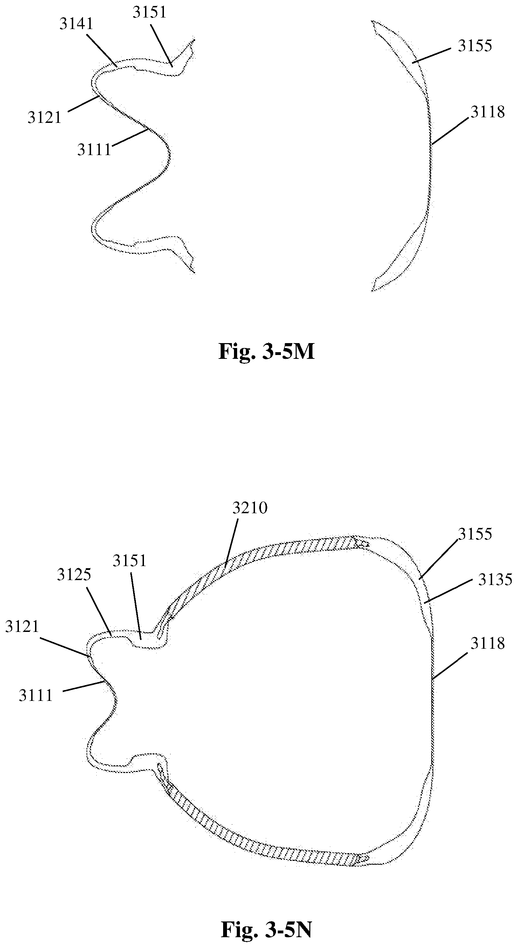

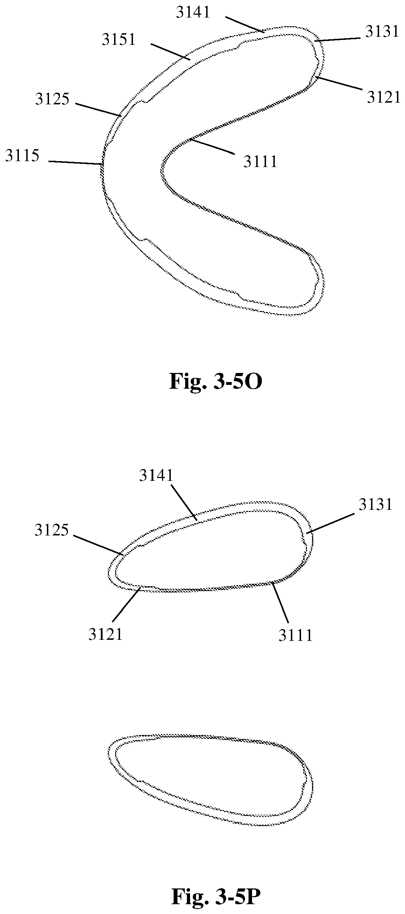

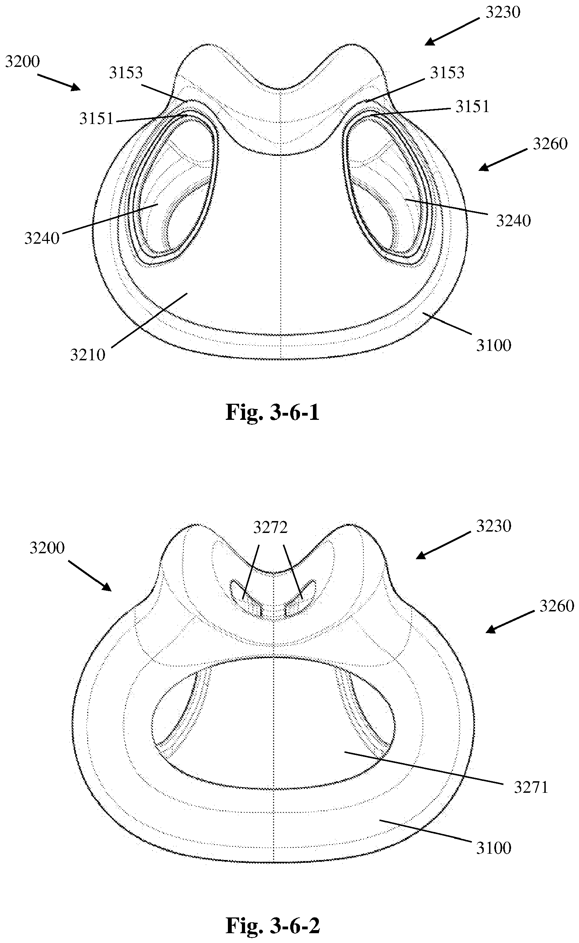

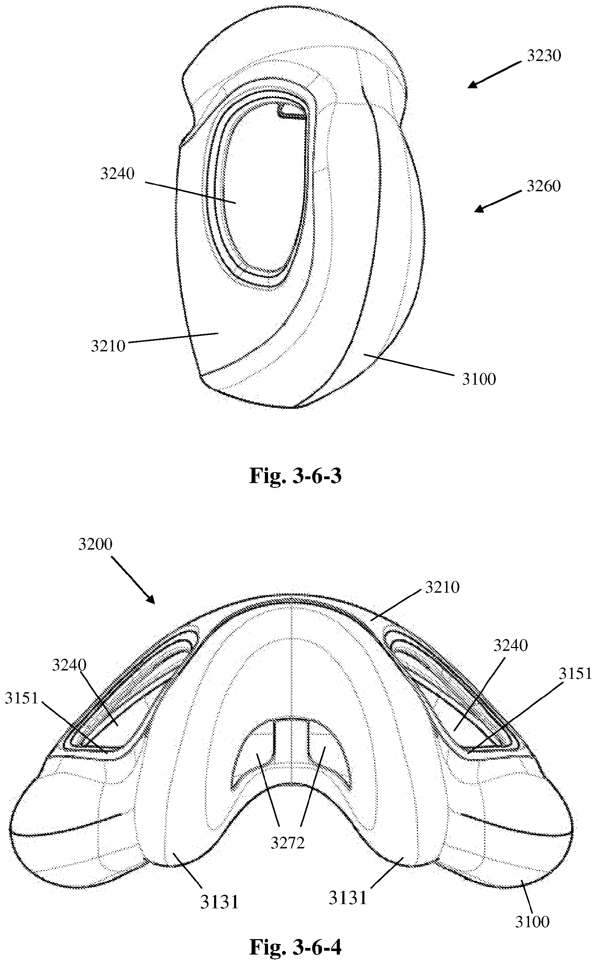

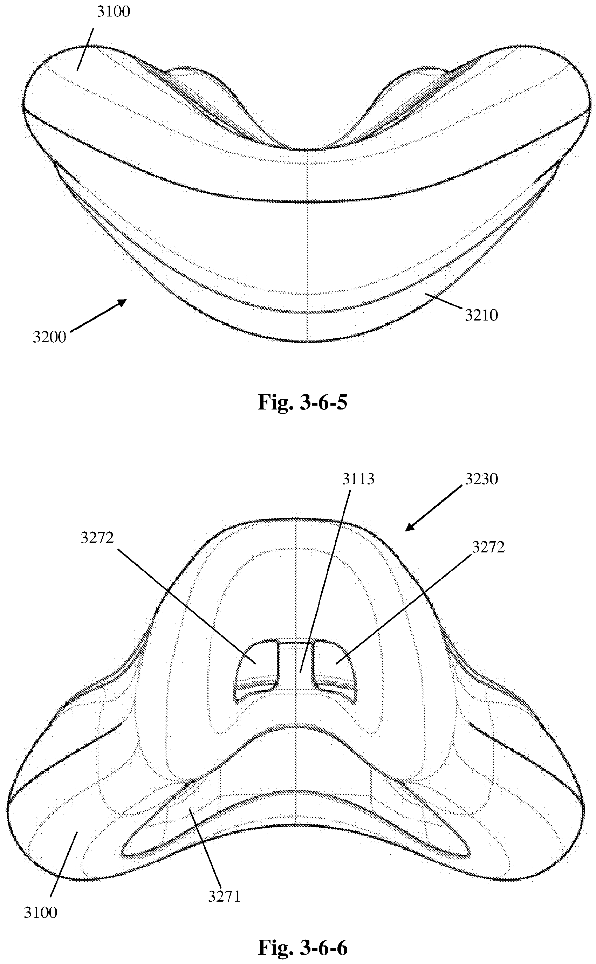

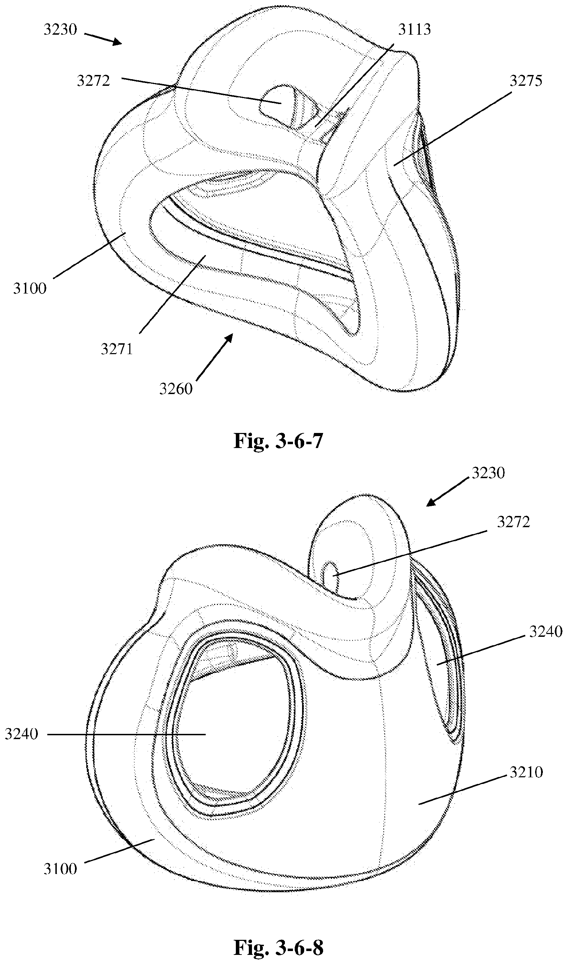

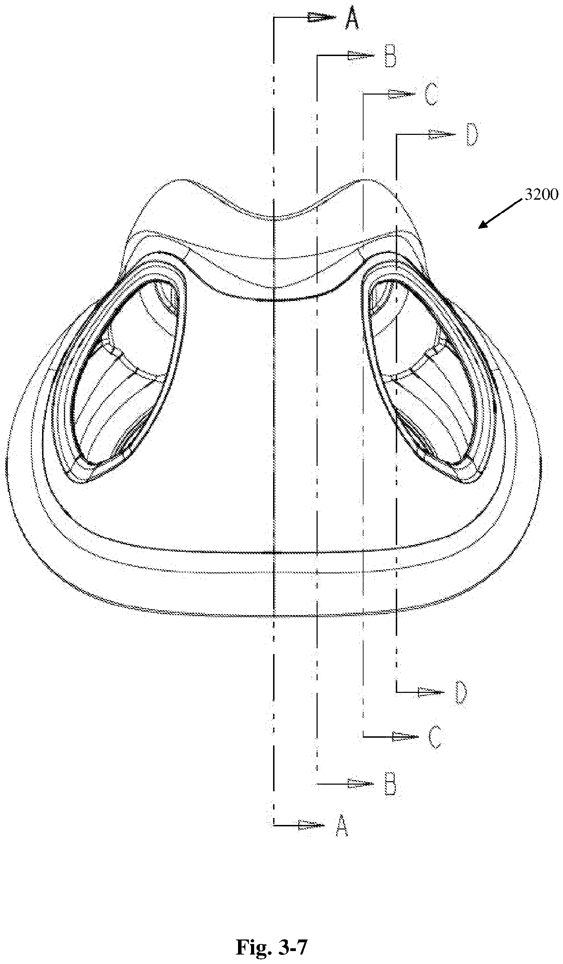

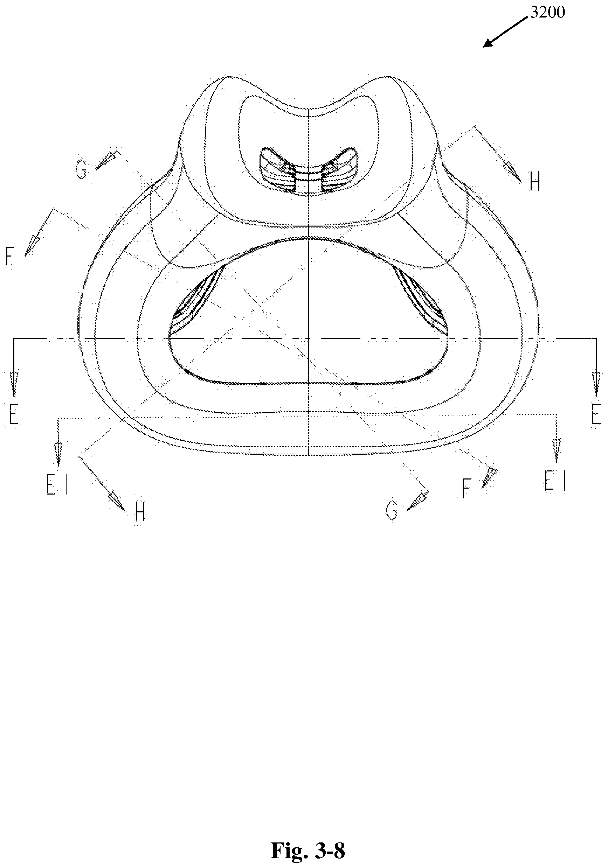

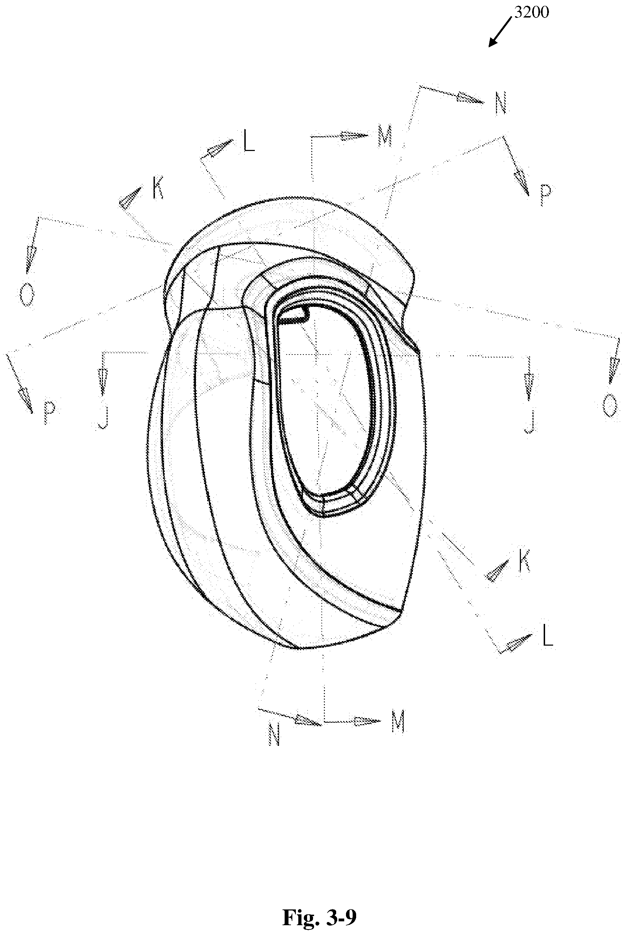

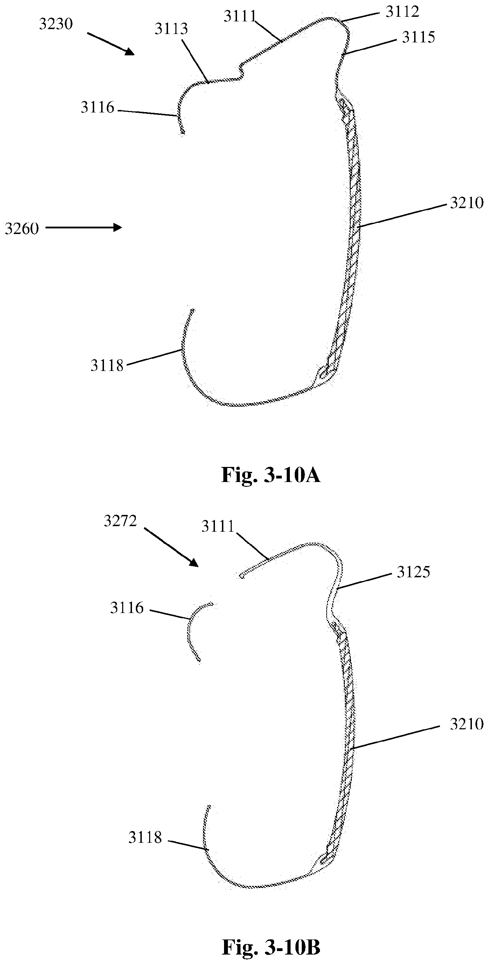

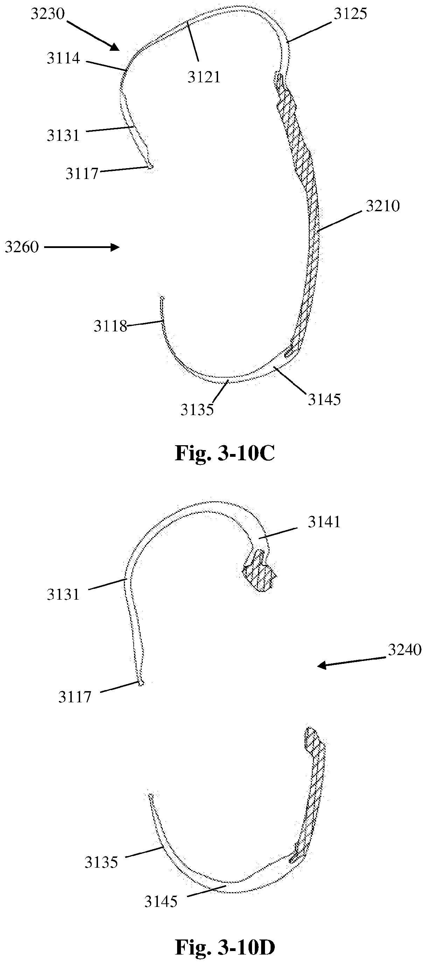

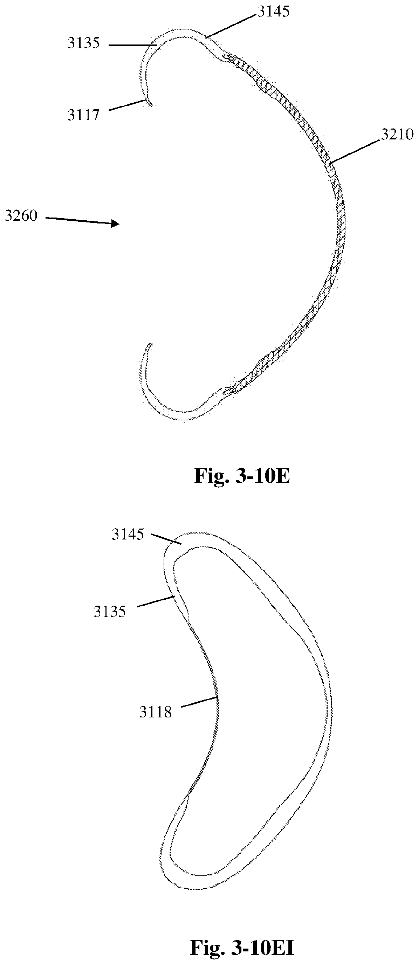

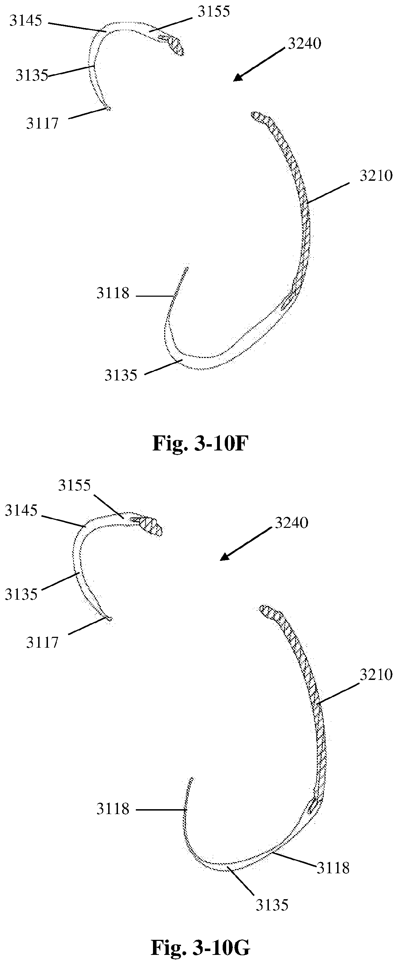

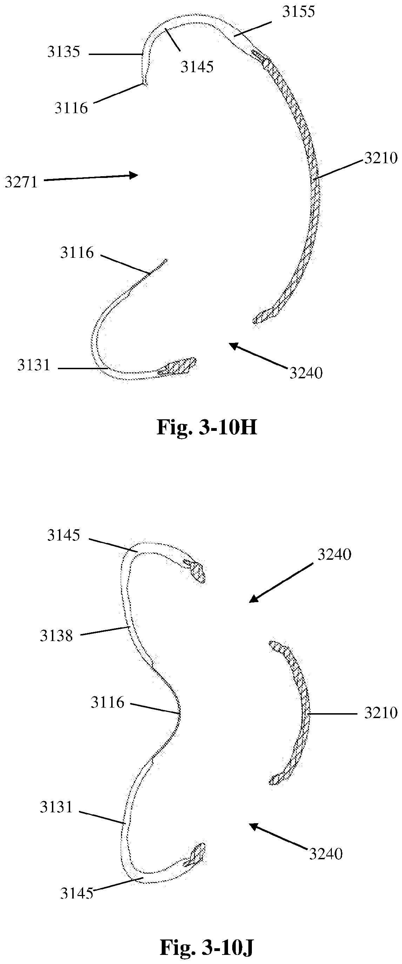

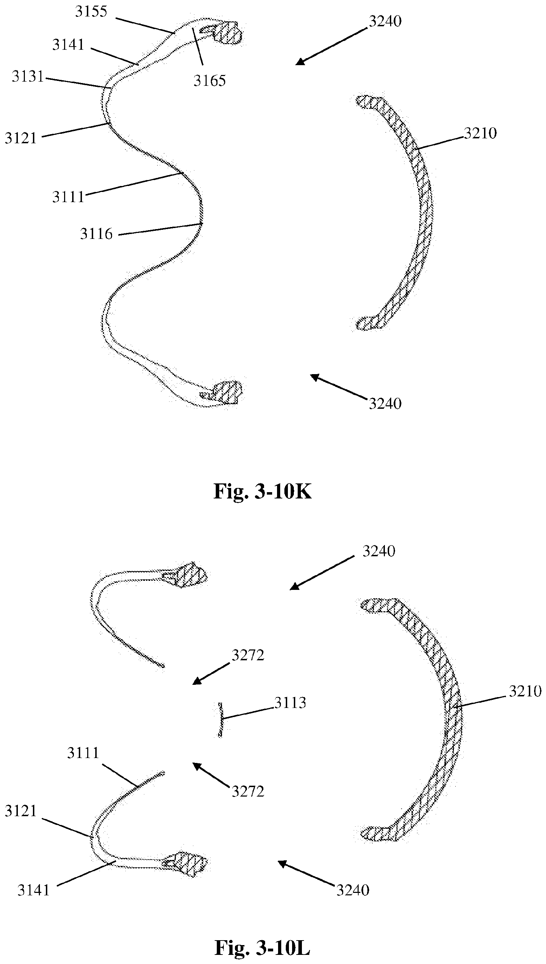

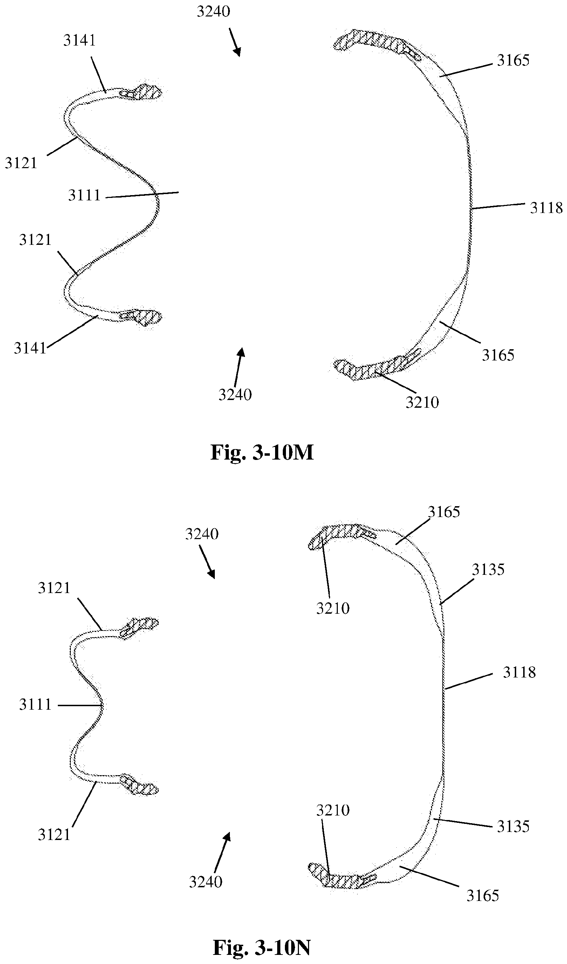

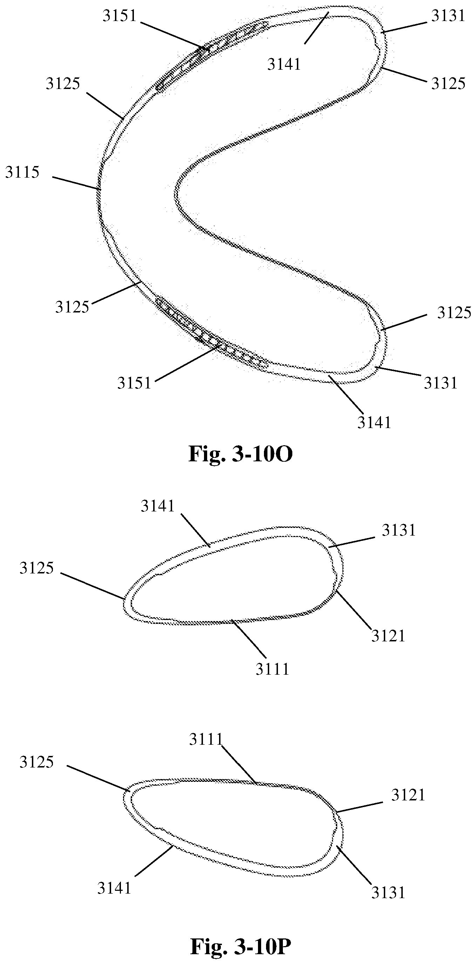

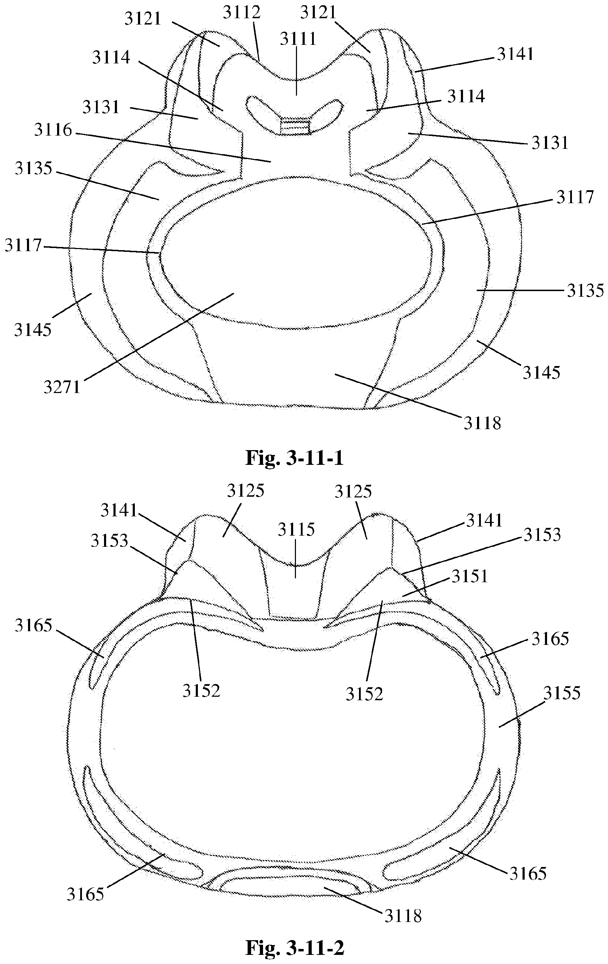

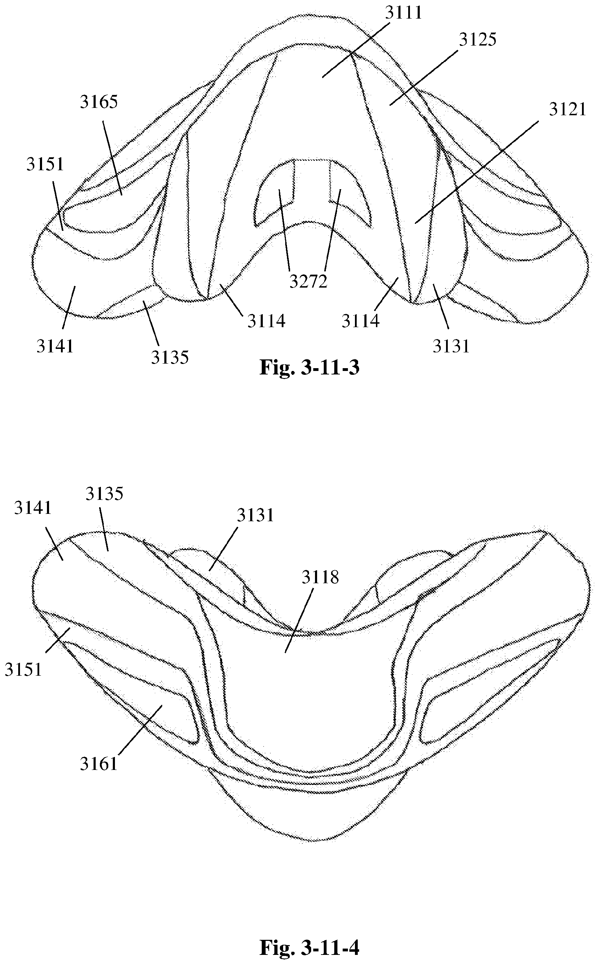

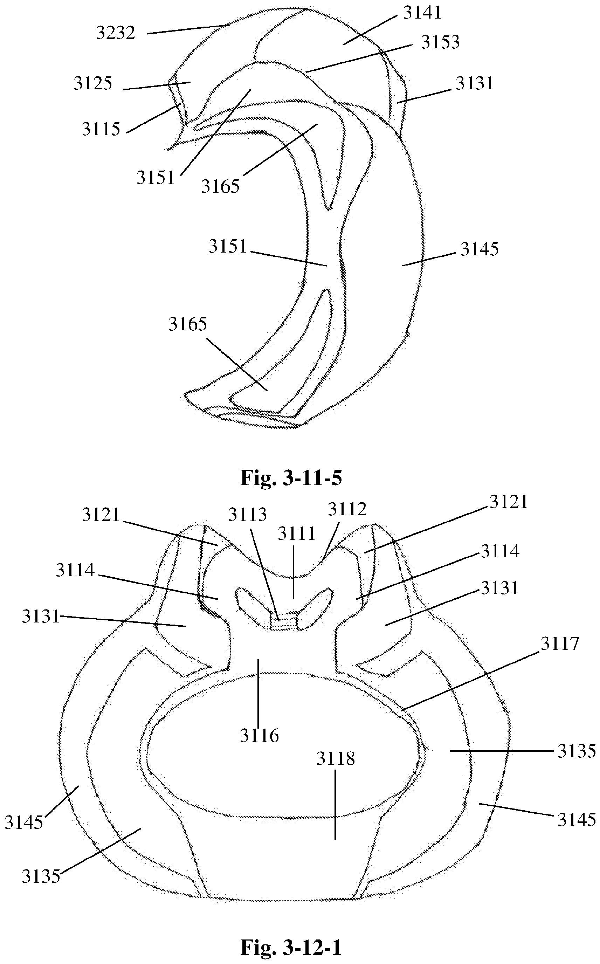

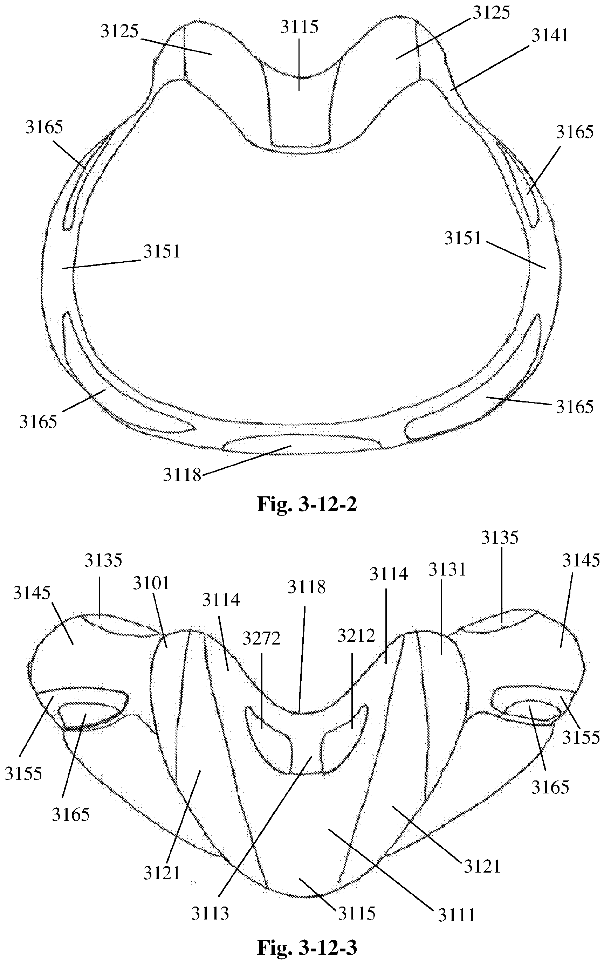

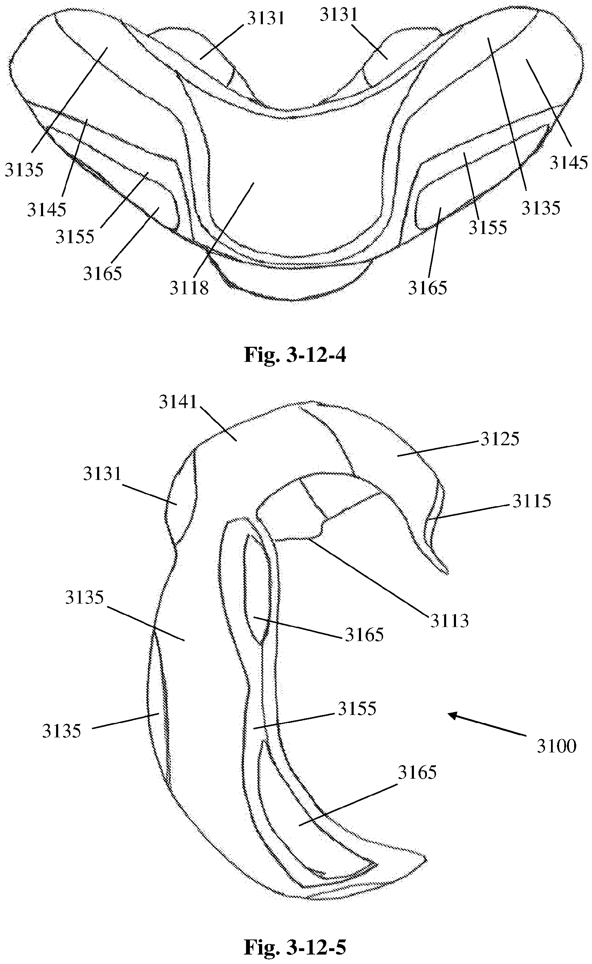

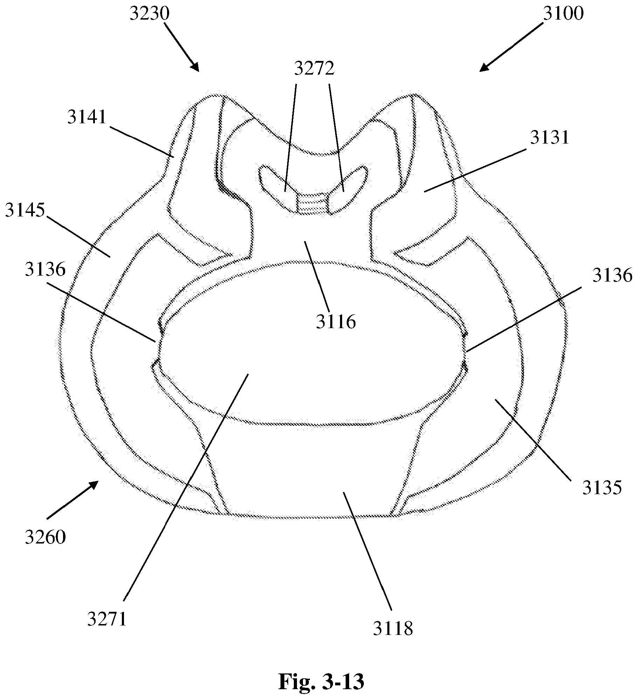

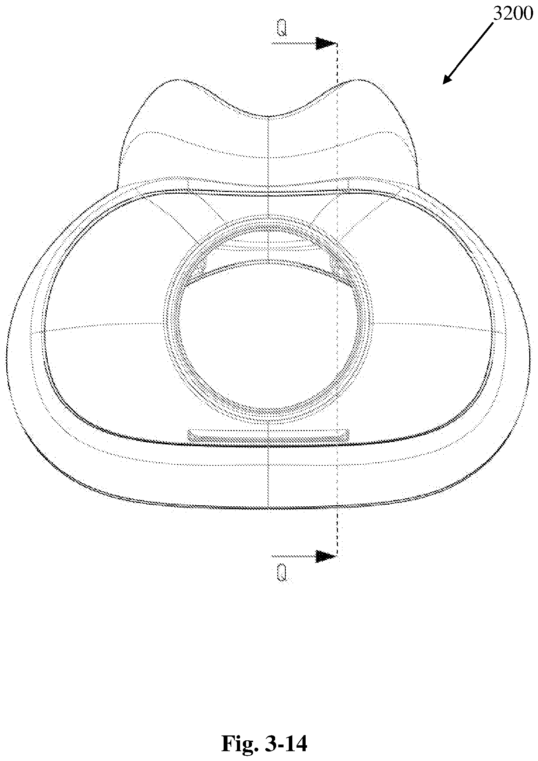

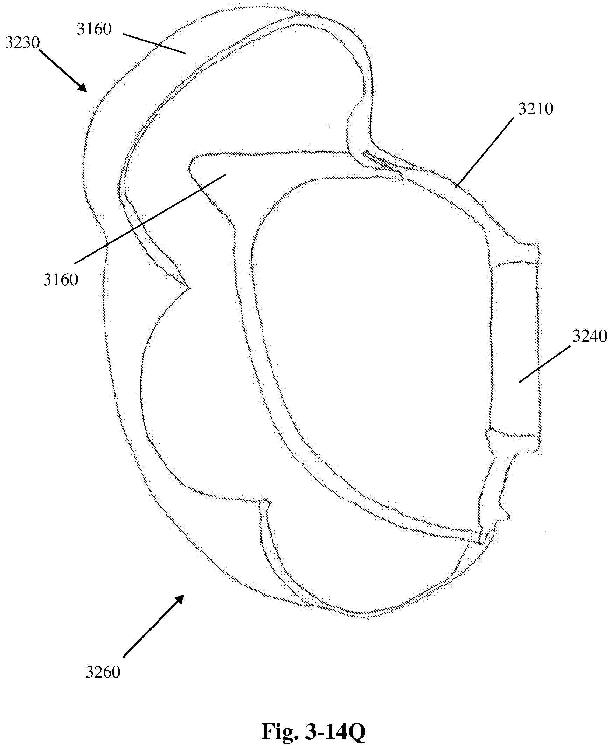

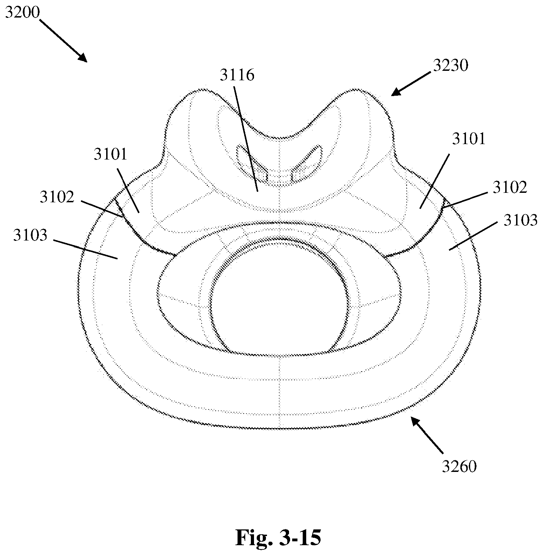

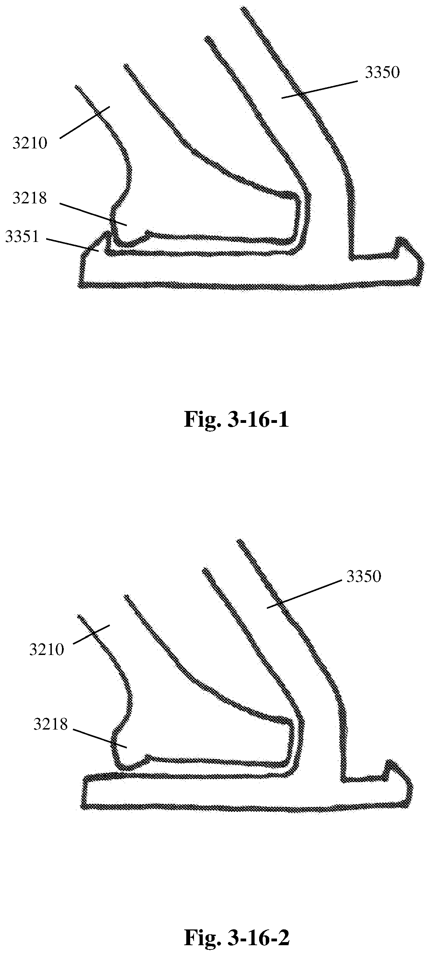

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.

Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

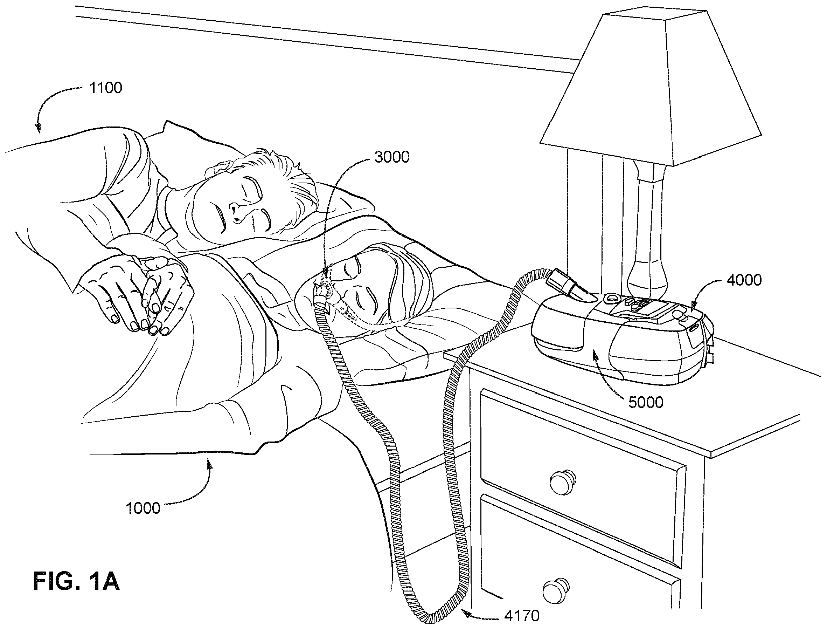

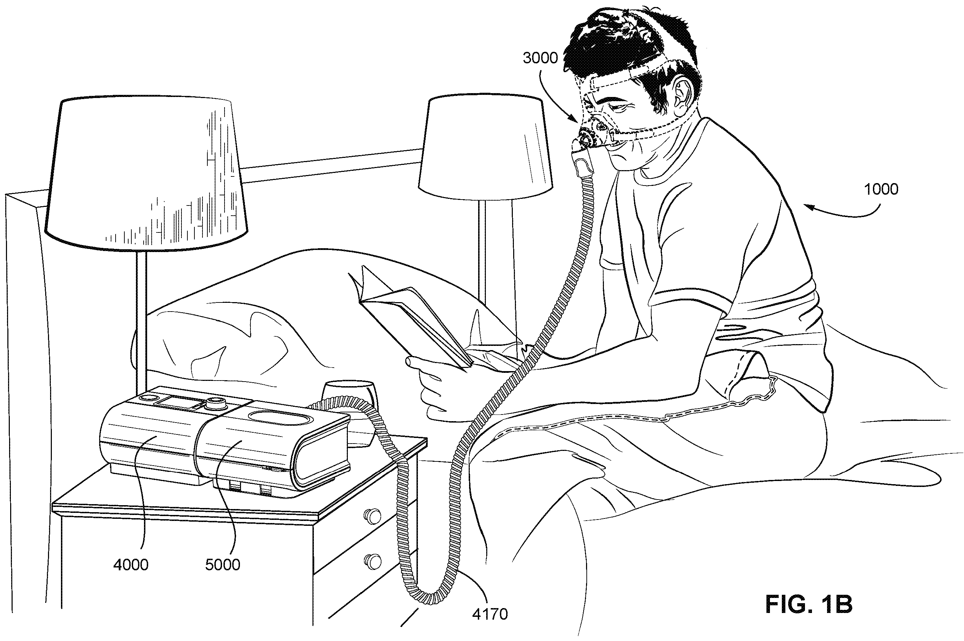

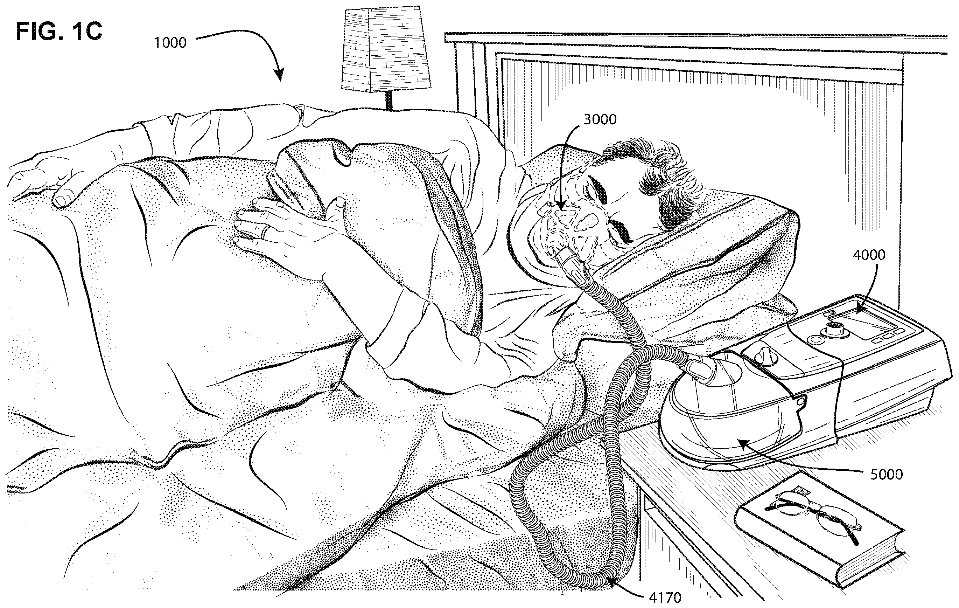

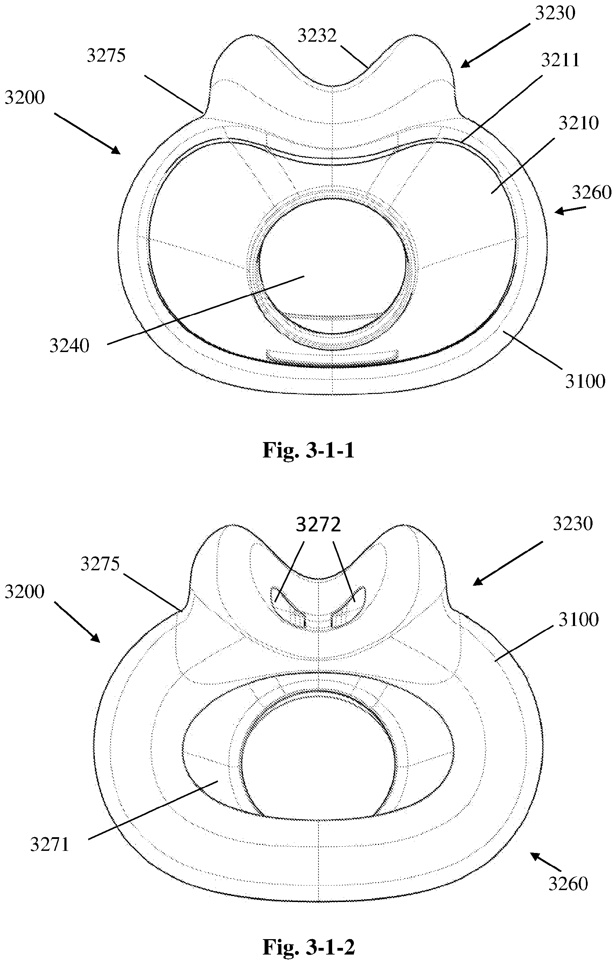

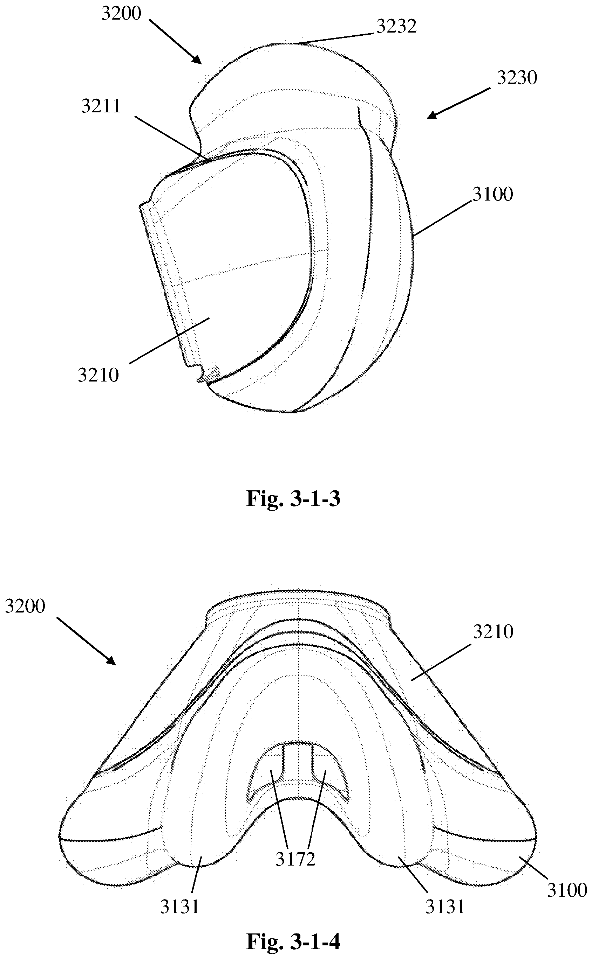

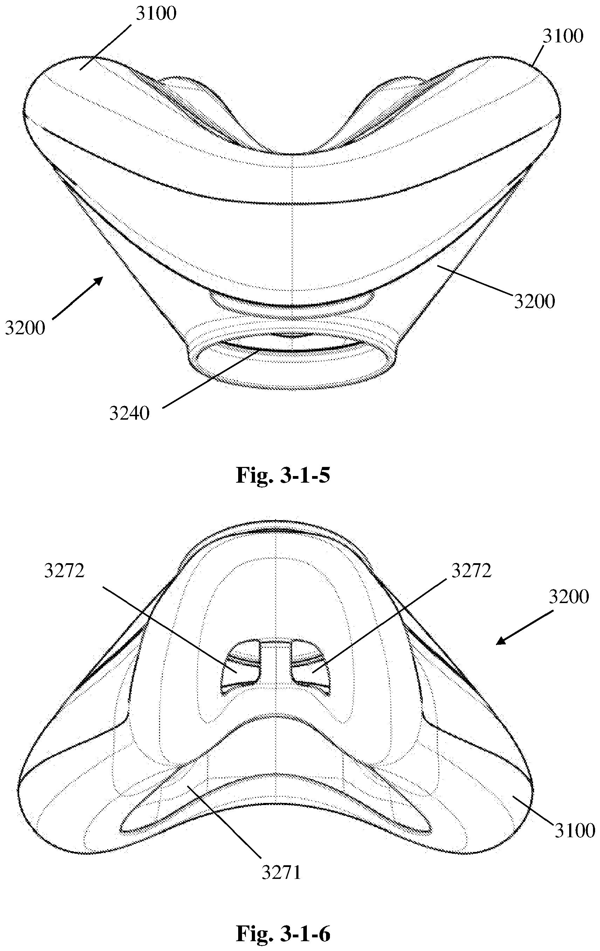

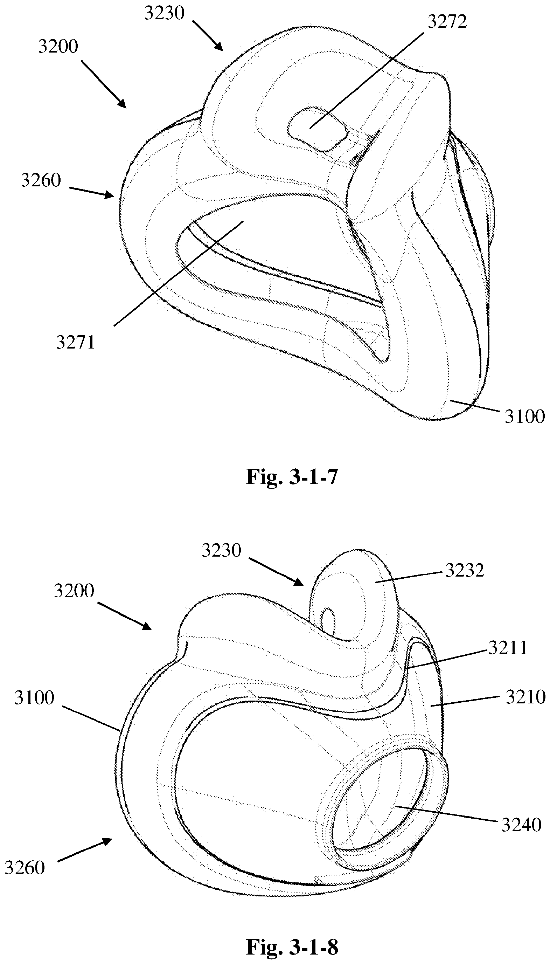

Fig. 3-1-1 is a front view of a plenum chamber 3200 in accordance with one form of the present technology.

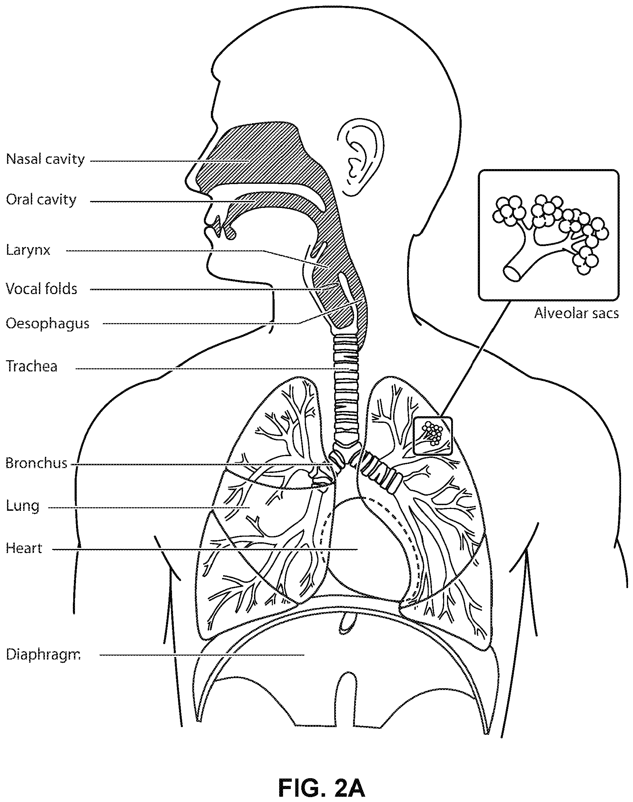

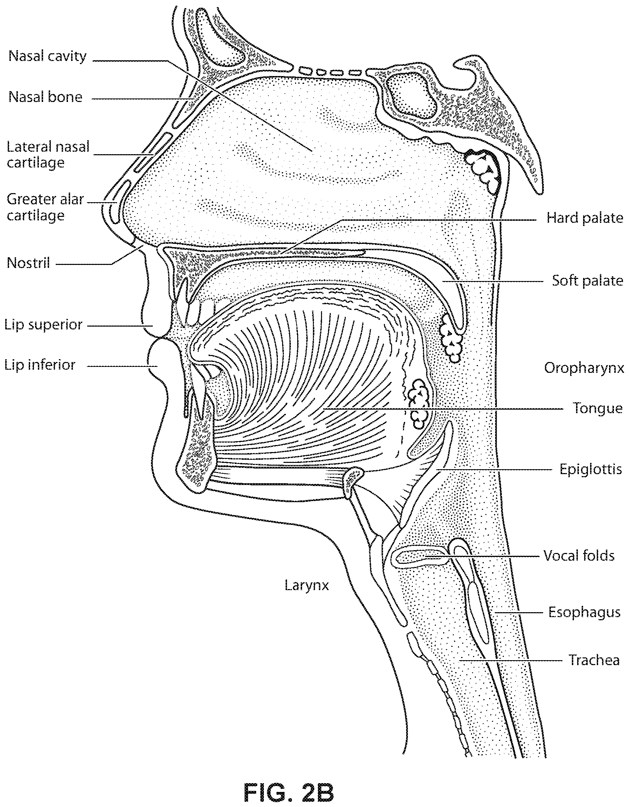

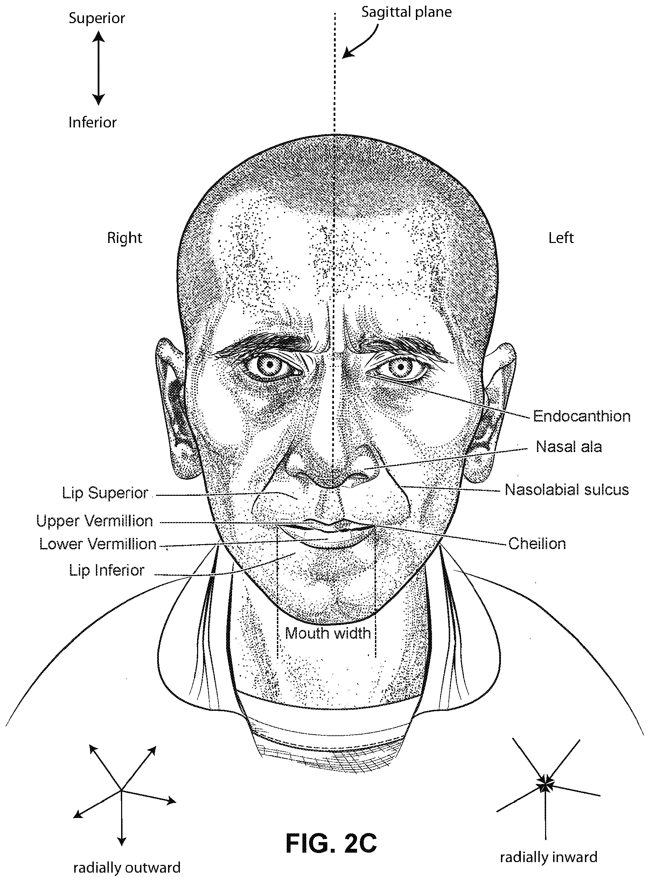

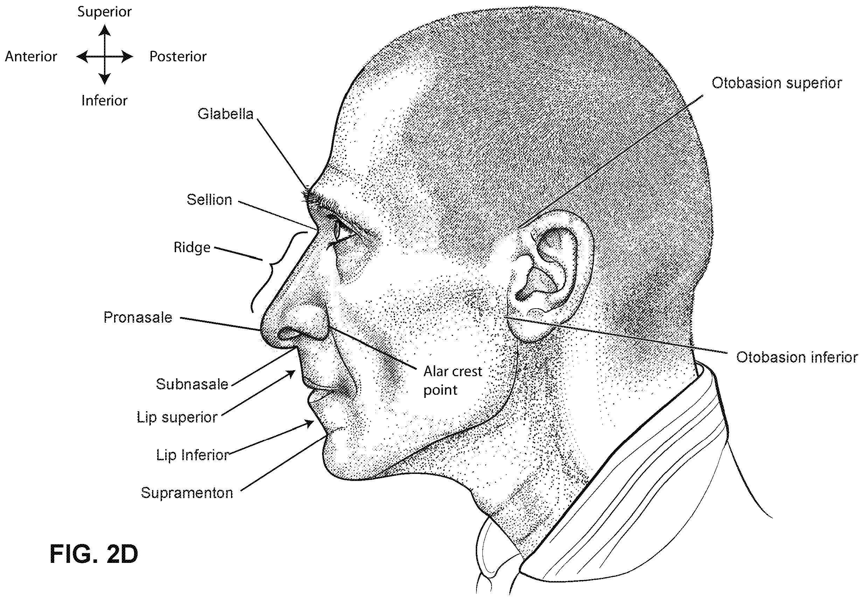

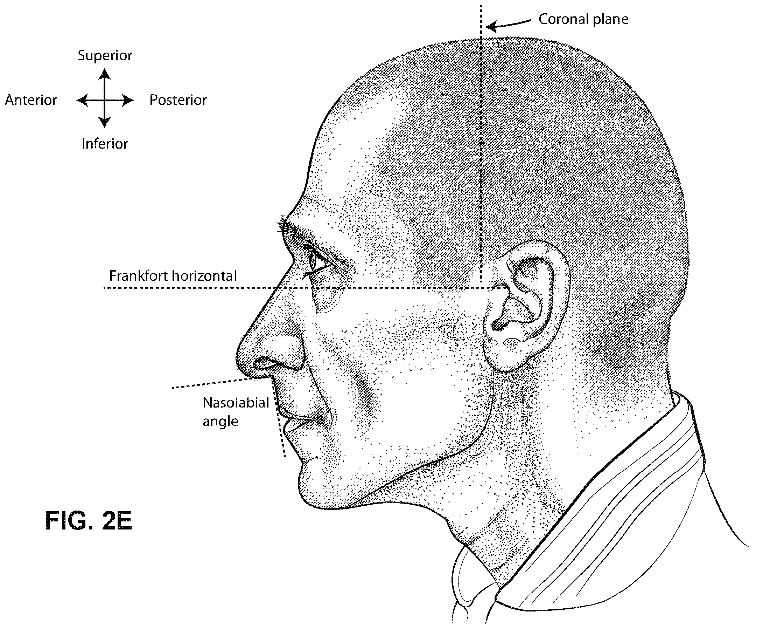

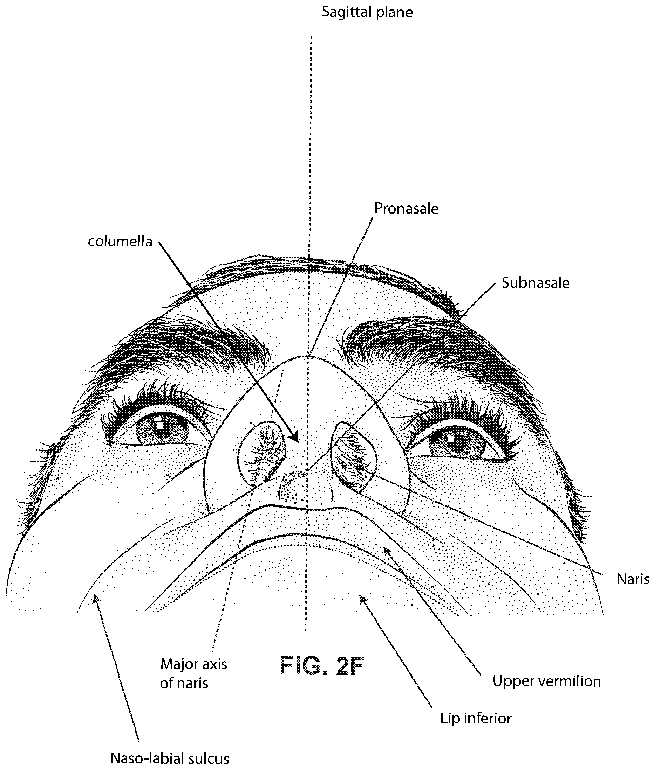

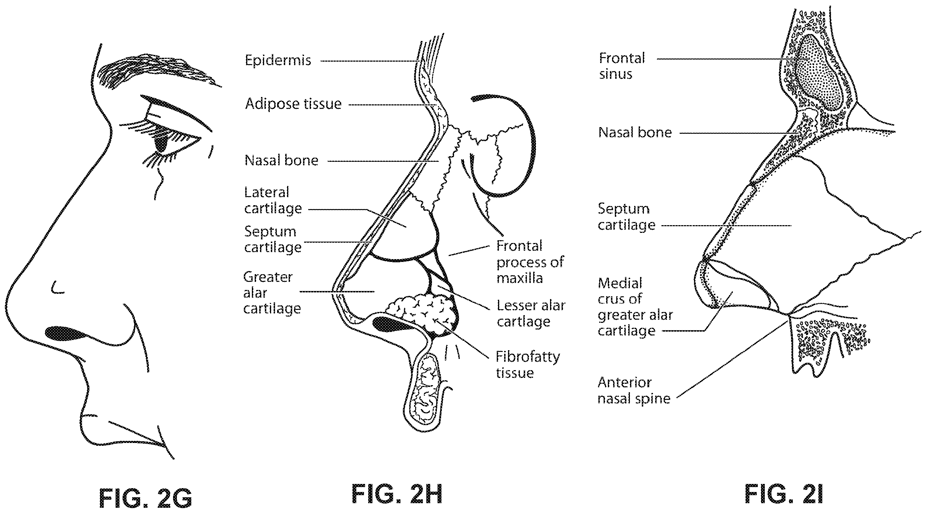

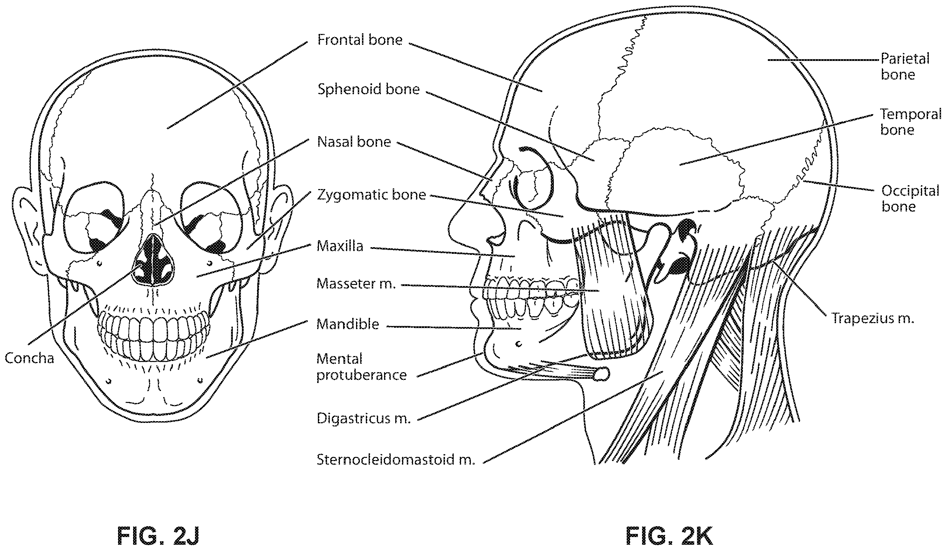

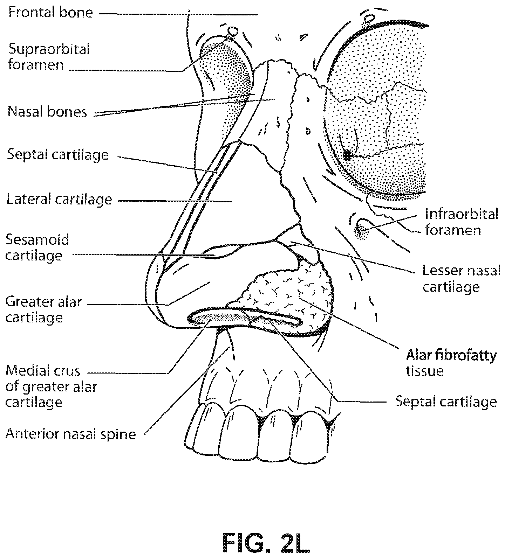

Fig 3-1-2 is a rear view of the plenum chamber 3200 of Fig. 3-1-1.

Fig 3-1-3 is a side view of the plenum chamber 3200 of Fig. 3-1-1.

Fig 3-1-4 is a top view of the plenum chamber 3200 of Fig. 3-1-1.

Fig 3-1-5 is a bottom view of the plenum chamber 3200 of Fig. 3-1-1.

Fig 3-1-6 is a perspective view of the plenum chamber 3200 of Fig. 3-1-1.

Fig 3-1-7 is another perspective view of the plenum chamber 3200 of Fig. 3-1-1.

Fig 3-1-8 is another perspective view of the plenum chamber 3200 of Fig. 3-1-1.

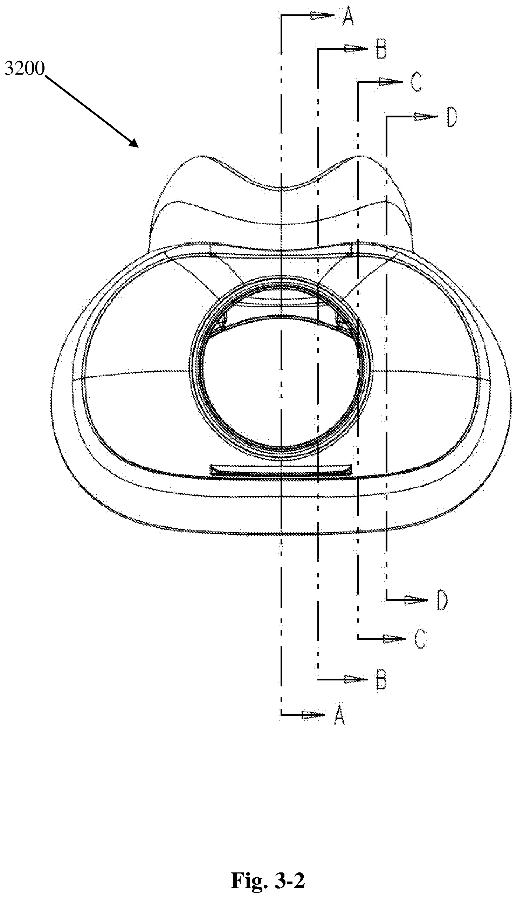

Fig. 3-2 is a front view of the plenum chamber 3200 of Fig. 3-1-1 with cross section lines A-A to D-D labelled.

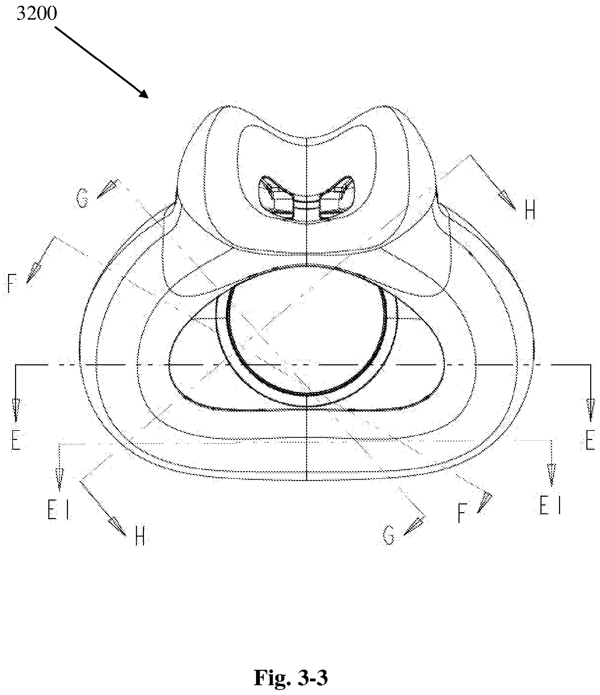

Fig. 3-3 is a rear view of the plenum chamber 3200 of Fig. 3-1-1 with cross section lines E-E to H-H labelled.

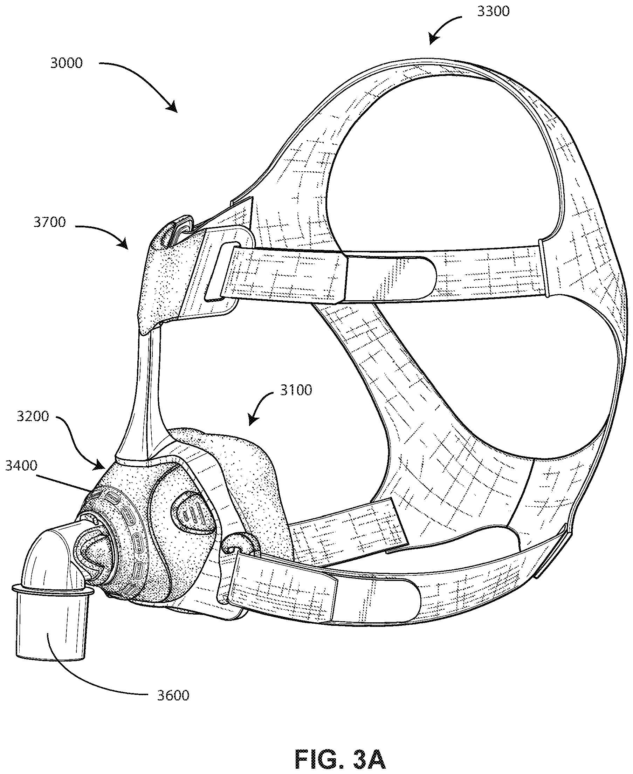

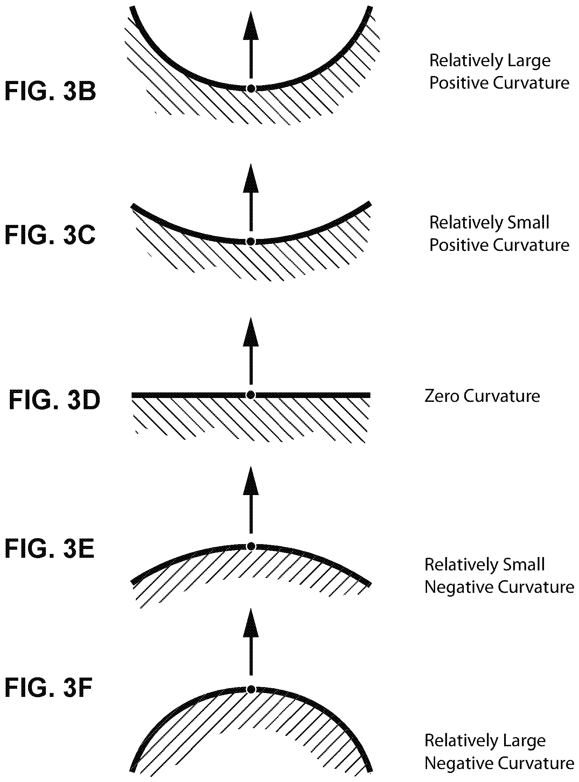

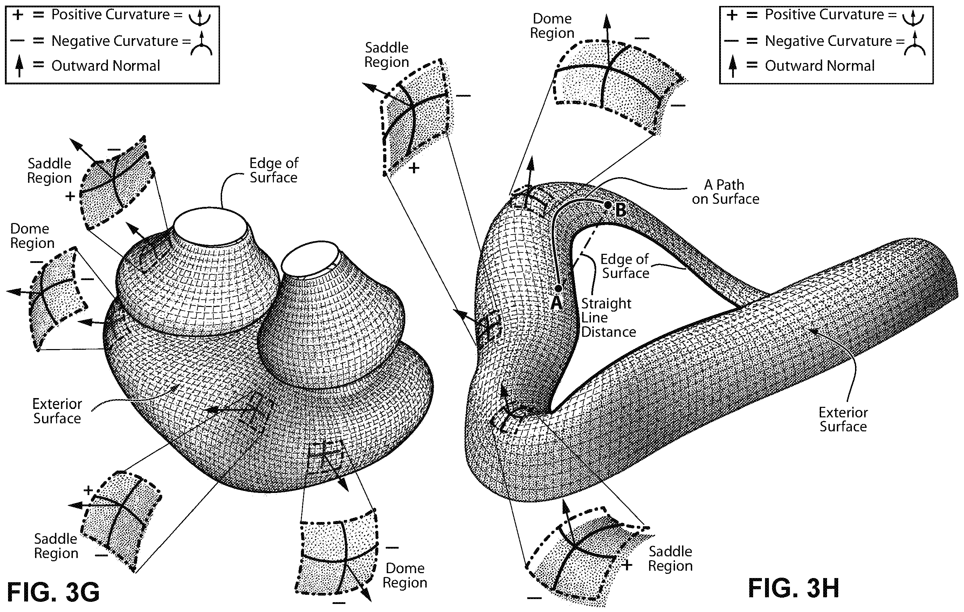

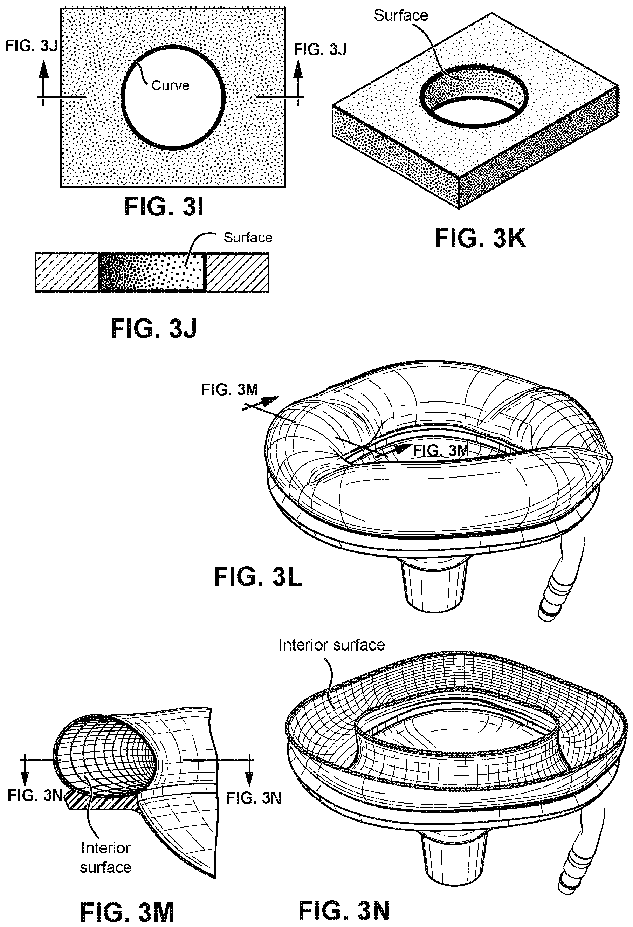

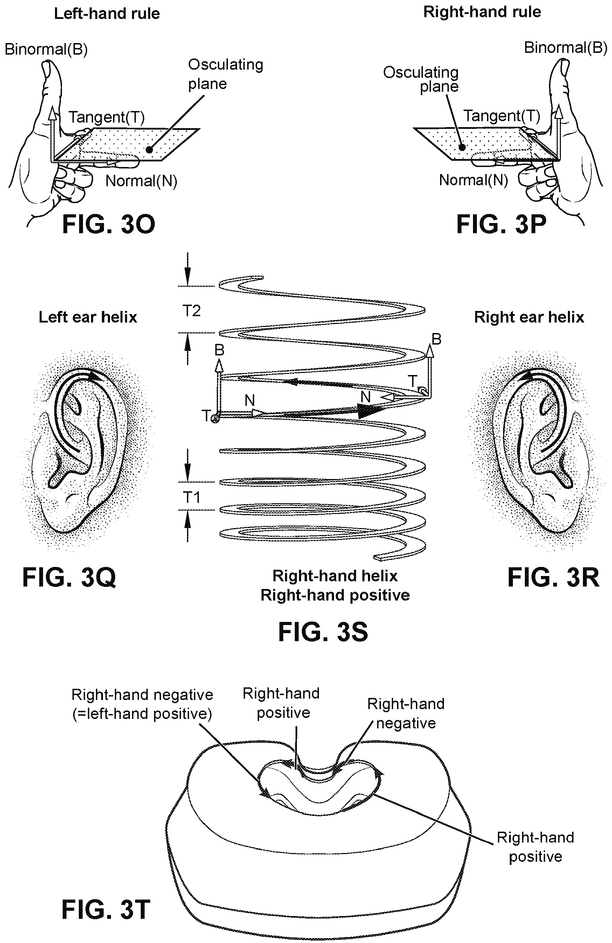

Fig. 3-4 is a side view of the plenum chamber 3200 of Fig. 3-1-1 with cross section lines J-J to P-P labelled.

Fig. 3-5A is a cross section view of the plenum chamber 3200 of Fig. 3-2 along the line A-A.

Fig. 3-5B is a cross section view of the plenum chamber 3200 of Fig. 3-2 along the line B-B.

Fig. 3-5C is a cross section view of the plenum chamber 3200 of Fig. 3-2 along the line C-C.

Fig. 3-5D is a cross section view of the plenum chamber 3200 of Fig. 3-2 along the line D-D.

Fig. 3-5E is a cross section view of the plenum chamber 3200 of Fig. 3-3 along the line E-E.

Fig. 3-5E1 is a cross section view of the plenum chamber 3200 of Fig. 3-3 along the line E1-E1.

Fig. 3-5F is a cross section view of the plenum chamber 3200 of Fig. 3-3 along the line F-F.

Fig. 3-5G is a cross section view of the plenum chamber 3200 of Fig. 3-3 along the line G-G.

Fig. 3-5H is a cross section view of the plenum chamber 3200 of Fig. 3-3 along the line H-H.

Fig. 3-5J is a cross section view of the plenum chamber 3200 of Fig. 3-4 along the line J-J.

Fig. 3-5K is a cross section view of the plenum chamber 3200 of Fig. 3-4 along the line K-K.

Fig. 3-5L is a cross section view of the plenum chamber 3200 of Fig. 3-4 along the line L-L.

Fig. 3-5M is a cross section view of the plenum chamber 3200 of Fig. 3-4 along the line M-M.

Fig. 3-5N is a cross section view of the plenum chamber 3200 of Fig. 3-4 along the line N-N.

Fig. 3-5O is a cross section view of the plenum chamber 3200 of Fig. 3-4 along the line O-O.

Fig. 3-5P is a cross section view of the plenum chamber 3200 of Fig. 3-4 along the line P-P.

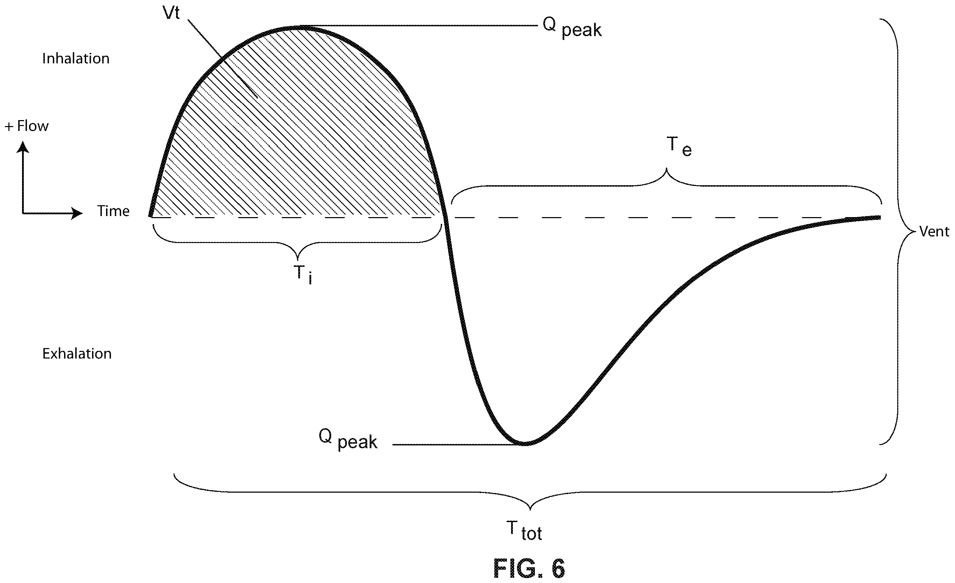

Fig. 3-6-1 is a front view of a plenum chamber 3200 in accordance with one form of the present technology.

Fig 3-6-2 is a rear view of the plenum chamber 3200 of Fig. 3-6-1.

Fig 3-6-3 is a side view of the plenum chamber 3200 of Fig. 3-6-1.

Fig 3-6-4 is a top view of the plenum chamber 3200 of Fig. 3-6-1.

Fig 3-6-5 is a bottom view of the plenum chamber 3200 of Fig. 3-6-1.

Fig 3-6-6 is a perspective view of the plenum chamber 3200 of Fig. 3-6-1.

Fig 3-6-7 is another perspective view of the plenum chamber 3200 of Fig. 3-6-1.

Fig 3-6-8 is another perspective view of the plenum chamber 3200 of Fig. 3-6-1.

Fig. 3-7 is a front view of the plenum chamber 3200 of Fig. 3-6-1 with cross section lines A-A to D-D labelled.

Fig. 3-8 is a rear view of the plenum chamber 3200 of Fig. 3-6-1 with cross section lines E-E to H-H labelled.

Fig. 3-9 is a side view of the plenum chamber 3200 of Fig. 3-6-1 with cross section lines J-J to P-P labelled.

Fig. 3-10A is a cross section view of the plenum chamber 3200 of Fig. 3-7 along the line A-A.

Fig. 3-10B is a cross section view of the plenum chamber 3200 of Fig. 3-7 along the line B-B.

Fig. 3-10C is a cross section view of the plenum chamber 3200 of Fig. 3-7 along the line C-C.

Fig. 3-10D is a cross section view of the plenum chamber 3200 of Fig. 3-7 along the line D-D.

Fig. 3-10E is a cross section view of the plenum chamber 3200 of Fig. 3-8 along the line E-E.

Fig. 3-10E1 is a cross section view of the plenum chamber 3200 of Fig. 3-8 along the line E1-E1.

Fig. 3-10F is a cross section view of the plenum chamber 3200 of Fig. 3-8 along the line F-F.

Fig. 3-10G is a cross section view of the plenum chamber 3200 of Fig. 3-8 along the line G-G.

Fig. 3-10H is a cross section view of the plenum chamber 3200 of Fig. 3-8 along the line H-H.

Fig. 3-10J is a cross section view of the plenum chamber 3200 of Fig. 3-9 along the line J-J.

Fig. 3-10K is a cross section view of the plenum chamber 3200 of Fig. 3-9 along the line K-K.

Fig. 3-10L is a cross section view of the plenum chamber 3200 of Fig. 3-9 along the line L-L.

Fig. 3-10M is a cross section view of the plenum chamber 3200 of Fig. 3-9 along the line M-M.

Fig. 3-10N is a cross section view of the plenum chamber 3200 of Fig. 3-9 along the line N-N.

Fig. 3-10O is a cross section view of the plenum chamber 3200 of Fig. 3-9 along the line O-O.

Fig. 3-10P is a cross section view of the plenum chamber 3200 of Fig. 3-9 along the line P-P.

Fig. 3-11-1 is a rear view of another plenum chamber 3200 having various portions identified.

Fig. 3-11-2 is a front view of the plenum chamber 3200 of Fig. 3-11-1 having various portions identified.

Fig. 3-11-3 is a top view of the plenum chamber 3200 of Fig. 3-11-1 having various portions identified.

Fig. 3-11-4 is a bottom view of the plenum chamber 3200 of Fig. 3-11-1 having various portions identified.

Fig. 3-11-5 is a side view of the plenum chamber 3200 of Fig. 3-11-1 having various portions identified.

Fig. 3-12-1 is a rear view of another plenum chamber 3200 in accordance with one form of the present technology having various portions identified.

Fig. 3-12-2 is a front view of the plenum chamber 3200 of Fig. 3-12-1 having various portions identified.

Fig. 3-12-3 is a top view of the plenum chamber 3200 of Fig. 3-12-1 having various portions identified.

Fig. 3-12-4 is a bottom view of the plenum chamber 3200 of Fig. 3-12-1 having various portions identified.

Fig. 3-12-5 is a side view of the plenum chamber 3200 of Fig. 3-12-1 having various portions identified.

Fig. 3-13 is a rear view of another plenum chamber 3200 in accordance with one form of the present technology having various portions identified.

Fig. 3-14 is a view of another plenum chamber 3200 in accordance with one form of the present technology.

Fig. 3-14Q is a cross section view of the plenum chamber 3200 of Fig. 3-14.

Fig. 3-15 is a rear view of another plenum chamber 3200 in accordance with one form of the present technology.

Fig. 3-16-1 is a cross section view of a connection between a plenum chamber 3200 and a frame 3350 in accordance with one form of the present technology.

Fig. 3-16-2 is another cross-section view of the connection between the plenum chamber 3200 and the frame 3350.

### 3.4 RPT DEVICE

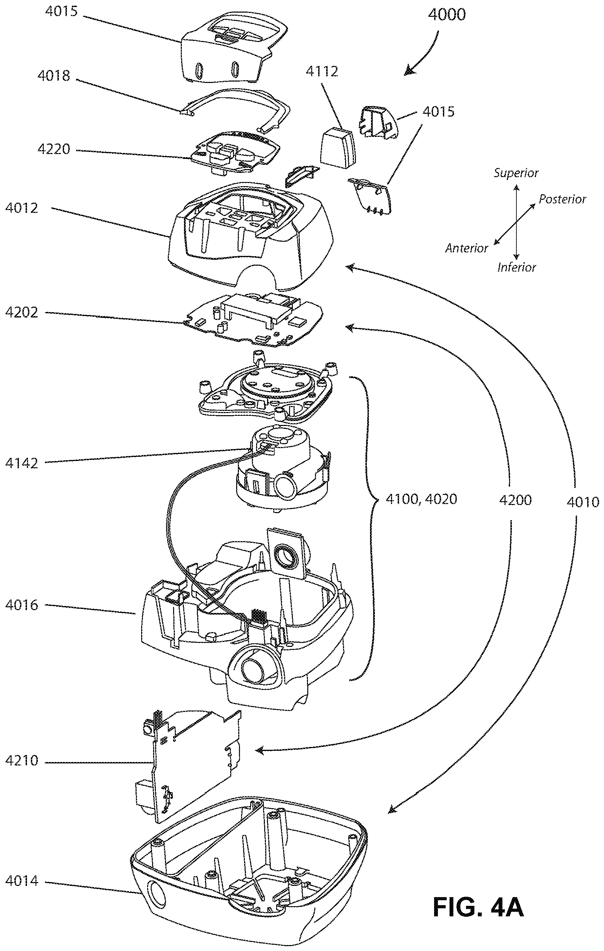

Fig. 4A shows an RPT device in accordance with one form of the present technology.

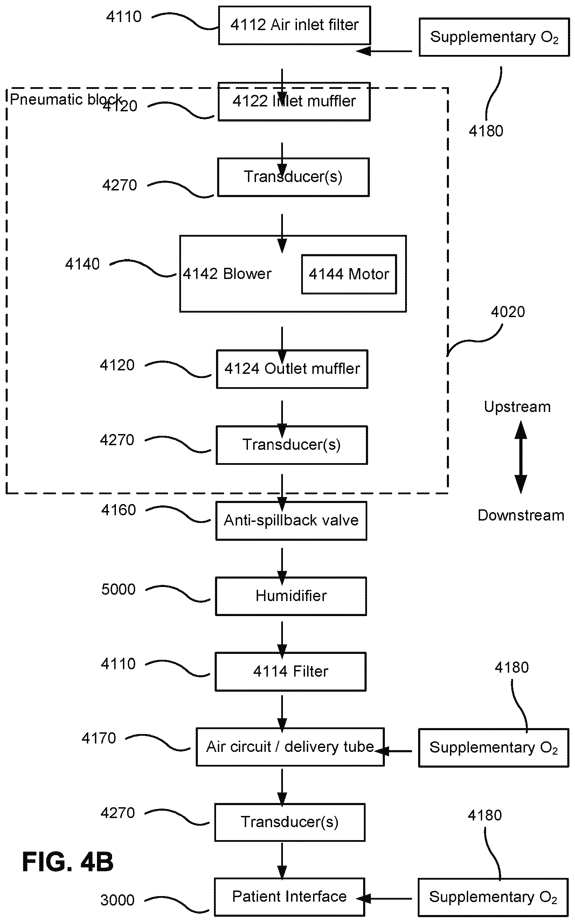

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 3.5 HUMIDIFIER

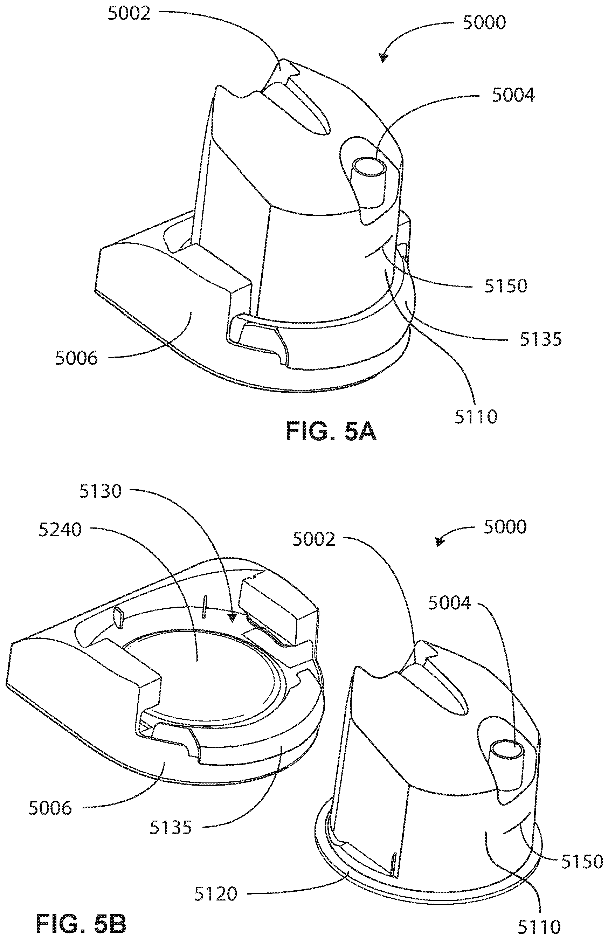

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

### 3.6 BREATHING WAVEFORMS

Fig. 6A shows a model typical breath waveform of a person while sleeping.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

Where anatomical directional terms are used in describing aspects and examples of the present technology, such as "anterior", "posterior", "superior", "inferior", "lateral", "medial" and the like, the directions are to be applied in the context of the present technology during use by a patient. For example, an anterior side of a patient interface refers to the side of the patient interface which is anterior with respect to the patient when the patient has donned the patient interface in the intended manner.

Where surfaces or portions are described as facing a direction, e.g. "superior-facing", "anterior-facing" and the like, unless the context clearly requires otherwise the surfaces or portions are to be understood as at least partially facing in the particular direction. A portion may be "superior-facing" if the portion generally faces a superior direction, even if it partially also faces another direction.

### 4.1 THERAPY

In one form, the present technology comprises a method for treating a respiratory disorder comprising the step of applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 TREATMENT SYSTEMS

In one form, the present technology comprises an apparatus or device for treating a respiratory disorder. The apparatus or device may comprise an RPT device 4000 for supplying pressurised air to the patient 1000 via an air circuit 4170 to a patient interface 3000.

### 4.3 PATIENT INTERFACE

A non-invasive patient interface 3000 in accordance with one aspect of the present technology comprises the following functional aspects: a plenum chamber 3200 comprising a seal-forming structure 3100, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

In some examples of the present technology, the plenum chamber 3200 is at least partially formed by a shell 3210 and the seal-forming structure 3100. The plenum chamber 3200 may also be known as a cushion module or cushion assembly, for example.

The patient interface 3000 in some examples of the technology is an oronasal patient interface. That is, the patient interface 3000 is configured to seal around both the patient's nasal airways and oral airway. In some examples the patient interface 3000 comprises separate seals around each of the nasal airways and oral airway. The patient interface 3000 may comprise a plenum chamber 3200 having a nasal portion 3230 and an oral portion 3260, as shown in Figs. 3-1-1 to 3-1-8 and Figs. 3-6-1 to 3-6-8 for example. The seal forming structure may be configured to surround the nasal airways at the nasal portion 3230 and to seal around the patient's mouth at the oral portion 3260. As such, the seal-forming structure may also be considered to have a nasal portion and an oral portion, the nasal portions and oral portions of the seal-forming structure comprising those parts that seal around the patient's nasal airways and mouth respectively.

In the examples shown in Figs. 3-1-1 to 3-6-8, the seal-forming structure 3100 at the nasal portion 3230 does not lie over a nose bridge region or nose ridge region of the patient's face and instead seals against inferior surfaces of the patient's nose. The nasal portion 3230 may seal against the lip superior, the ala and the anterior surface of the pronasale and/or the inferior surface of the pronasale. The actual sealing locations may differ between patients. The nasal portion 3230 may also be configured to contact and/or seal to a region of the patient's face between the ala and the nasolabial sulcus and at the lateral portions of the lip superior proximate the nasolabial sulcus.

The seal-forming structure 3100 of the oral portion 3260 may be configured to form a seal to a periphery of the patient's mouth in use. The oral portion 3260 may be configured to form a seal to the patient's face at the lip superior, nasolabial sulcus, cheeks, lip inferior, supramenton, for example.

The seal-forming structure 3100 may have one or more holes therein such that the flow of air at a therapeutic pressure is delivered to the patient's nares and to the patient's mouth via the one or more holes. The seal-forming structure may define an oral hole and one or more nasal holes to deliver the flow of air to the patient. In the examples shown in Figs. 3-1-1 to 3-1-8 and 3-6-1 to 3-6-8, the plenum chamber 3200 comprises a seal-forming structure 3100 comprising an oral hole 3271 and two nasal holes 3272. Each of the nasal holes 3272 may be positioned on the plenum chamber 3200 to be substantially aligned with a nare of the patient in order to deliver a flow of air thereto in use.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 4.3.1 Plenum chamber

The plenum chamber 3200 has a perimeter that is shaped to be complementary to the surface contour of the face of an average person in the region where a seal will form in use. In the examples shown in Figs. 3-1-1 to 3-1-8 and 3-6-1 to 3-6-8, the plenum chamber comprises a shell 3210 and a seal-forming structure 3100. In these examples a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, a portion of the plenum chamber 3200 is constructed from a transparent material, e.g. a transparent polycarbonate. In the examples shown in Figs. 3-1-1 to 3-1-8 and 3-6-1 to 3-6-8, the shell 3210 is constructed from transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

Figs. 3-1-1 to 3-15 show plenum chambers 3200 according to examples of the present technology which are formed partially by a shell 3210. Additionally, the plenum chamber 3200 is formed partially by the seal-forming structure 3100. In some examples the seal-forming structure 3100 is overmoulded to the shell 3210. The seal-forming structure 3100 may alternatively be formed separately from the shell 3210 and be configured to permanently or removably connect to the shell 3210. The seal-forming structure 3100 and shell 3210 are integrally formed.

In this example the shell 3210 is formed from polycarbonate and the seal-forming structure 3100 is formed from silicone. The silicone may have a hardness of 30 or 40 Durometer. A silicone or similar material with this hardness is advantageous for comfort and flexibility to conform and seal to the patient's face. The use of polycarbonate (or other stiffer material), with its higher hardness and stiffness than silicone is advantageous in providing portions of higher resistance to deformation with less material than would be required to provide the same resistance with silicone. Using less material can be advantageous in keeping overall bulk and weight down, which may reduce obtrusiveness to the user. In alternative examples the shell 3210 may be formed from nylon or polypropylene. The seal-forming structure 3100 may alternatively be formed from a suitable foam or any suitable thermoplastic elastomer.

As shown in Figs. 3-14 and 3-14Q, the shell 3210 may have posteriorly protruding portions 3215 at superior lateral corners of the shell 3210. The posteriorly protruding portions 3215 may reinforce a base of the nasal portion and may help provide stability to the seal-forming structure 3100. The posteriorly protruding portions 3215 also help reinforce the locations of the seal-forming structure proximate posterior corners 3131 (shown in Figs. 3-1-4 and 3-6-4, among others) which also support the nasal portion of the plenum chamber on the patient's face, and also advantageously fit into the creases proximate the patient's ala and the nasolabial sulcus. The posterior pointing portions 3215 may protrude from the shell 3210 towards the location of the patient's nasolabial sulcus or the patient's cheeks alongside the nasolabial sulcus. The posterior pointing portions may be provided inferior to lateral support portions 3151 (described below).

### 4.3.1.1 Lateral Support Portions

In some examples of the present technology, the plenum chamber 3200 comprises lateral support portions 3151 on the anterior side of the nasal portion 3230 of the plenum chamber 3200, as shown in Figs. 3-5C, 3-5D, 3-5O and 3-11-1 to 3-11-5, among others. The lateral support portions may have a higher resistance to deformation than one or more adjacent portions of the seal-forming structure 3100. The lateral support portions 3151 may be stiffer than regions of the plenum chamber 3200 superior to lateral support portions 3151. Additionally, or alternatively, the lateral support portions 3151 may be stiffer than a central region of the plenum chamber 3200. The regions of relatively greater stiffness may be in the form of fins configured to provide areas of relatively high rigidity in comparison to surrounding areas of the plenum chamber. The seal-forming structure 3100 shown in Figs. 3-11-1 to 3-11-5 comprises lateral support portions 3151 therein. Additionally, the plenum chamber 3200 shown in Figs. 3-6-1 to 3-6-8 comprises lateral support portions 3151 in the shell 3210.

The lateral support portions 3151 may assist in providing lateral stability to the nasal portion 3230 of the plenum chamber 3200. In the examples of Figs. 3-1-1 to 3-11-5 the lateral support portions 3151 are provided to laterally-spaced, at least partially anterior-facing sides of the of the plenum chamber 3200. In particular, the lateral support portions 3151 are provided to the non-patient facing side (e.g. anterior side or at least partially anterior-facing sides) of the nasal portion 3230, in this example one lateral support portion 3151 is provided on each lateral side of the plenum chamber 3200 to an at least partially anterior-facing wall. The plenum chamber 3200 is configured so that the lateral support portions 3151 are positioned in the seal-forming structure 3100 approximately opposite the patient's ala in use.

The lateral support portions 3151 may comprise regions of the plenum chamber 3200 having an increased material thickness than surrounding or adjacent areas. Alternatively, or additionally, the lateral support portions 3151 may be formed by a material stiffer than material in surrounding or adjacent areas.

The lateral support portions 3151 may be substantially fin shaped (e.g. having a curved superior boundary and a flatter inferior boundary). As shown in Figs. 3-11-1 to 3-11-5, the lateral support portions 3151 of the seal-forming structure 3100 comprise curved superior boundaries 3153 and flatter inferior boundaries 3152. Similarly, the lateral support portions 3151 of the shell 3210 as shown in Figs 3-6-1 to 3-6-8 have curved superior boundaries 3152 (although in this example there are no distinct inferior boundaries of the lateral support portions 3151). A fin shape, in particular the provision of a curved superior boundary or edge, is advantageous as the superior edge or boundary of the lateral support portion 3151 follows the curvature of the superior periphery 3232 of the nasal portion 3230. This provides a consistent height of the nasal portion 3230 above the shell 3210, which may be useful for providing consistent or controlled stiffness to the structure of the nasal portion 3230 (as discussed below). Additionally, the curved superior boundaries 3153, rather than a flat boundary around the entire anterior side of the nasal portion 3230, mean that the central anterior-facing portion of the nasal portion remains flexible to avoid excessive force on the patient's pronasale.

The lateral support portions 3151 may control collapsibility of the anterior side of the plenum chamber 3200. The height (e.g. amount of superior extension) of the lateral support portions may be selected to balance collapsibility with structural rigidity. A certain amount of flexibility in the structure of the seal-forming structure 3100 is desirable as it enables the seal-forming structure 3100 to accommodate a wide range of nose shapes and sizes. However, if the lateral support portions 3151 extend too far in the superior direction, the seal-forming structure 3100 may not be accommodating enough or may not be sufficiently comfortable. Alternatively, if the lateral support portions 3151 do not extend far enough in the superior direction, then the seal-forming structure 3100 may be so prone to collapse that it may not be able to prevent blowout.

Additionally, some flexibility in the overall structure of the seal-forming structure 3100 can be advantageous for accommodating long and/or narrow noses. When the seal-forming structure 3100 is moved upwards into contact with the underside of a narrow nose, an amount of flexibility in the overall structure of the nasal portion 3230 is desirable as it enables the sides of the seal-forming structure 3100 to be drawn inwards as a result of downwards force applied to the centre of the seal-forming structure 3100 by the patient's nose. If there is insufficient flexibility in the sides of the seal-forming structure 3100, the seal-forming structure 3100 may be less accommodating to narrow noses and/or may be uncomfortable if the patient is required to tighten the headgear to compensate. Excess flexibility may result in the seal-forming structure 3100 being incapable of holding its shape and maintaining an effective seal.

The height of the lateral support portions 3151, should be appropriate to provide sufficient structural rigidity to the nasal portion 3230 while remaining flexible enough to enable the seal-forming structure 3100 to seal comfortably to a wide range of noses. The lateral support portions 3151 may extend superiorly approximately 35-65% of the height of the nasal portion 3230, such as 40-60% or 50%, in examples, of the distance between a base of the nasal portion 3230 on an anterior side of the plenum chamber 3200 and the most superior point of the plenum chamber 3200.

In other examples, the lateral support portions 3151 could be substituted by other stiffening/rigidising structures or features. In some examples, ribs are provided to the interior or the exterior of the nasal portion 3230 of the seal-forming structure 3100 generally in the locations of the lateral support portions 3151, to provide structural rigidity to the nasal portion 3230. In other examples, the lateral support portions 3151 may be rigidised. The seal-forming structure 3100 may comprise supportive inserts (e.g. rigidiser elements) provided to the lateral support portions 3151. In one example, the seal-forming structure 3100 may be overmoulded to one or more rigidiser elements to provide rigidity to the lateral support portions 3151.

In other examples the plenum chamber 3200 comprises an undercushion that provides the necessary support to the structure of the nasal portion 3230. The undercushion may be thicker than the face-contacting portions of the seal-forming structure 3100, allowing the patient-contacting walls to be thin for comfort and able to conform to the patient's nose and face.

Further still, in some examples a separate component is provided to support the structure of the nasal portion 3230 of the seal-forming structure 3100. For example, a frame to which the plenum chamber 3200 connects to has portions that reinforce the seal-forming structure 3100 in the areas of the lateral support portions 3151.

### 4.3.1.1.1 Lateral support portions formed by seal-forming structure

The plenum chamber 3200 shown in Figs. 3-1-1 to 3-5P and 3-11-1 to 3-11-5 comprises lateral support portions 3151 provided by the seal-forming structure 3100. The lateral support portions 3151 are provided to the anterior side of the plenum chamber 3200 and on each lateral side of the nasal portion 3230. In this example the lateral support portions 3151 are provided at inferior regions of each lateral side of the nasal portion 3230.

At least a majority of the nasal portion 3230 is formed by the seal-forming structure 3100 in this example. The majority of the nasal portion 3230 of the plenum chamber 3200 is formed from a soft, flexible resilient material. The majority of the nasal portion 3230 is formed from silicone in this example. In the examples of Figs. 3-1-1 to 3-5P, substantially the entire nasal portion is formed by the seal-forming structure 3100. In the examples of Figs. 3-6-1 to 3-10P, portions of the nasal portion are formed by the shell 3210.

In this example, the lateral support portions 3151 are regions of the seal-forming structure 3100 having relatively greater stiffness in comparison to one or more adjacent regions of the seal-forming structure 3100. In particular, the lateral support portions 3151 have a greater material thickness than regions of the seal-forming structure 3100 superior to the lateral support portions 3151. This greater material thickness provides an amount of stiffness or structural rigidity to the structure of the seal-forming structure 3100 and in particular the nasal portion 3230. Much of the posterior side of the nasal portion 3230 has a low wall thickness for comfort and ability to seal against the patient's face, which may not provide significant structural rigidity to the shape of the nasal portion 3230. The lateral support portions 3151 compensate for the lack of structural rigidity provided by the patient-contacting walls and increase the overall structural rigidity to the nasal portion 3200.

As shown in Figs. 3-11-2 and 3-11-5, the lateral support portions 3151 each comprise a substantially flat inferior boundary 3152. While the substantially flat inferior boundaries 3152 are approximately flat, they may still have a small amount of curvature, for example due to some curvature being required for the base of the nasal portion to transition into the oral portion 3230 of the plenum chamber 3200. Each flat inferior boundary 3152 may be adjacent a respective posteriorly protruding portion 3215 of the shell 3210. The lateral support portions 3151 each comprise a curved superior boundary 3153 in this example. Additionally, the curved superior boundaries 3153 may have a curvature which substantially matches or follows curvature of a superior periphery 3232 of the seal-forming structure 3100 at the nasal portion 3230.

### 4.3.1.1.2 Lateral support portions formed by shell

The plenum chamber 3200 shown in Figs. 3-6-1 to 3-10P comprises lateral support portions 3151 provided by the shell 3210 of the plenum chamber 3200. The lateral support portions 3151 in this example comprise superior portions of the shell 3210. The lateral support portions 3151 extend in a superior direction into the nasal portion 3230 of the plenum chamber 3200. The lateral support portions 3151 comprise portions of the shell 3210 provided to the nasal portion 3210 in this example. While the shell 3210 has a generally flat top edge in the example show in Figs. 3-1-1 to 3-5P, the shell 3210 in the example shown in Figs. 3-6-1 to 3-10P comprises a curved top edge 3211 which comprises lateral support portions 3151 that extend in a superior direction to a greater extent than a central portion of the top edge 3211, forming the two lateral support portions 3151. Each of the lateral support portions 3115 in this example comprises a curved superior edge or boundary. The curvature of each superior edge substantially matches or follows a curvature of a superior periphery 3232 of the seal-forming structure. The lateral support portions 3151 of the shell 3210 occupy regions of the anterior and lateral facing sides of the nasal portion 3230 approximately opposite from the patient's nasal ala in use.

The lateral support portions 3151 in the examples shown in Figs. 3-6-1 to 3-10P provide similar functions to the lateral support portions 3151 shown in the examples of Figs. 3-1-1 to 3-5P. The lateral support portions 3151 provide structural rigidity to the nasal portion 3230 of the seal-forming structure 3100. Since the patient-contacting (posterior) side of the seal-forming structure 3100 comprises relatively low thickness, flexible walls, the lateral support portions 3151 provide a level of structural rigidity to the nasal portion 3230.

### 4.3.1.2 Plenum chamber inlet port

The shell 3210 may comprise one or more plenum chamber inlet ports 3240. The one or more plenum chamber inlet ports 3240 may allow for a connection to other components, such as a frame, decoupling structure, vent arrangement, heat and moisture exchanger (HMX), constant-flow vent (CFV), anti-asphyxia valve (AAV) and/or connection port to a conduit in various examples.

In the example shown in Figs. 3-1-1 to 3-1-8, the plenum chamber 3200 comprises a single inlet port 3240. The inlet port 3240 is provided substantially centrally in the shell 3210. The inlet port 3240 in this example may be configured to connect to a frame to which headgear or other positioning and stabilising structure components can be connected. The inlet port 3240 is substantially circular in this exemplary form of the technology.

In the example shown in Figs. 3-6-1 to 3-6-8, the plenum chamber comprises two inlet ports 3240. The inlet ports 3240 are provided to lateral sides of the shell 3210. The inlet ports 3240 in this example are configured to connect to conduits which connect to a decoupling component located above the patient's head where the conduits are connected to the air circuit. The conduits may form part of the positioning and stabilising structure 3300, i.e. they may be "headgear conduits". In some examples the inlet ports 3240 may receive combined headgear and conduit connection assemblies, in order to provide multiple functions such as venting, supply of the flow of air and headgear attachment points. The combined headgear and conduit connection assemblies may also comprise an AAV. The inlet ports 3240 in this example are approximately elliptically shaped, e.g. oval.

The superior periphery of each inlet port 3240 in the examples shown in Figs. 3-6-1 to 3-6-8 is formed by a lateral support portion of the nasal portion 3230. In examples where the inlet ports 3240 connect to headgear conduits, this advantageously enables the connections of the plenum chamber 3200 to the headgear conduits to be made in superior positions of the plenum chamber 3200. This may enable shorter conduits, more favourable force vectors and/or a smaller conduit footprint over the patient's face, in comparison to inlet ports 3240 provided at inferior positions of the plenum chamber 3200.

In one embodiment, the plenum chamber 3200 connects to a frame and is supported in front of the patient's face via a positioning and stabilising structure (e.g. headgear). The connection to the frame may be a snap fit connection. Alternatively, the connection may be a press fit, bayonet connection or other suitable connection.

In one example the frame 3350 comprises snap fit hooks 3351 that fit over a rim of the shell 3210. In some examples two snap fit hooks 3351 provided to the frame snap over a rim 3218 provided to the air inlet port 3240 of the plenum chamber 3200. Figure 3-16-1 is a cross section view of the connection between the frame 3350 and the plenum chamber 3200 in a horizontal plane, showing horizontal snap fit hooks 3351 snapped over the rim 3218 of the air inlet port 3240. In this example, the two snap fit hooks 3351 are horizontally opposed across the air inlet port 3240. In other examples the arms are vertically opposed. Providing horizontally opposed snap fit arms (e.g. at 9 o'clock and 3 o'clock positions around the air inlet 3240) may advantageously provide resistance to disengagement when lateral forces are exerted on the plenum chamber 3200 or frame 3350. Figure 3-16-2 is a cross section view of the connection between the frame 3350 and the plenum chamber 3200 in a vertical plane, showing a lack of snap fit connections at the vertically opposed sides of the connection. Since the patient may sleep with their head on its side, the plenum chamber 3200 may be more likely to receive lateral forces in use than vertical forces. Providing horizontally opposed snap fit connections means that the patient can rotate the plenum chamber 3200 upwards or downwards with respect to the frame 3320 to disassemble it, but that lateral forces on the plenum chamber 3200 during use may be unlikely to disengage plenum chamber 3200 from the frame.

### 4.3.1.3 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs - the actual sealing surface - may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 4.3.1.3.1 Sealing mechanisms

In one form, the seal-forming structure 3100 includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.3.2 Nasal region

In certain forms of the present technology, the seal-forming structure 3100 comprises a central portion configured to form a seal to inferior surfaces of the patient's nose. The central portion may seal to an inferior periphery of the patient's nose (e.g. surrounding the patient's nares and to the patient's lip superior).

As shown in Figs. 3-11-1 to 3-12-5, the seal-forming structure 3100 comprises a central portion configured to seal to an inferior periphery of the patient's nose in use and intermediate portions configured to be located against or proximate the ala of the patient's nose in use. More particularly, the central portion includes a superior-facing central portion 3111 and an anterior-facing central portion 3115. Most or all of the contact of the central portion with the patient's nose is made by the superior-facing central portion 3111. Additionally, the intermediate portions comprise a superior-facing intermediate portion 3121 and an anterior facing intermediate portion 3125. Most or all of the contact of the intermediate portion with the patient's nose is made by the superior-facing intermediate portion 3121.

A majority of the seal formed by the seal-forming surface 3100 to the inferior periphery of the patient's nose may be made by the superior-facing central portion 3111 and lip superior portion 3118. The inferior surfaces of the patient's nose and lip superior can have complex geometry and can also be very sensitive to pressure. Accordingly, it is advantageous for the areas of the plenum chamber 3200 that will contact or seal against these locations to be flexible and compliant, to avoid exerting excessive pressure on the face at these regions. The low stiffness, which in this embodiment is provided by a thin wall thickness in the central portion of the nasal portion 3230 of the seal-forming structure 3100 proximate the nasal holes 3272, enable the cushion to readily deform to seal against the surfaces on the underside of the patient's nose, e.g. against the pronasale towards the anterior direction, the nasal ala on either lateral side and the lip superior. The low wall thickness of the superior-facing central portion 3111 and lip superior portion 3118 also enables the seal-forming structure to inflate in these portions to conform to the geometry of inferior surfaces and periphery of the patient's nose. The thin wall of the seal-forming structure 3100 in the superior-facing central portion 3111 and lip superior portion 3116 is advantageous in creating a good seal to the complex geometry under and around the nose. The thin wall can deform and inflate under pressure, conforming to the surfaces of the patient's face to create an effective and yet comfortable seal.

The super-facing central region 3111 superior and anterior to the nasal holes 3272 of the seal-forming structure is intended to seal against the inferior and partially anterior surfaces of the patient's pronasale. This area of the seal-forming structure 3100 may be provided with a low wall thickness because the pronasale can be a relatively sensitive area to many patients. In fact, the central portion extends from a superior-facing central portion 3111 on the posterior (i.e. patient facing) side of the cushion over a central saddle portion 3112 and peripheral edge and into an anterior-facing central portion 3115 on the anterior (i.e. non-patient facing) side of the seal-forming structure. A thin wall thickness in this region avoids exerting excessive pressure on the sensitive pronasale area.

A lip superior portion 3116 of the seal-forming structure 3100 is intended to seal against the lip superior. The lip superior portion 3116 is provided centrally and inferior and posterior to the nasal holes 3272. The lip superior portion 3116 may comprise a low wall stiffness. In some examples the low wall stiffness is provided by a low wall thickness. Similarly to the area of the seal-forming structure 3100 intended to seal against the pronasale, a low wall thickness extends over the central inferior/posterior region of the nasal portion 3230 of the seal-forming structure 3100 because the lip superior can be a sensitive area. The low wall thickness may exert lower forces on the lip superior than would be exerted by a relatively thick wall thickness.

While there is an advantage for comfort in keeping the wall thickness low in the regions configured to contact the sensitive pronasale and lip superior regions, in examples of the present technology the wall thickness in these regions is not reduced to the extent that the seal-forming structure 3100 is no longer able to maintain a stable seal to the patient's face. If the wall thickness is too low in these regions, the seal-forming structure could be prone to creasing, which can break the seal and create a leak path through which air can flow to the surroundings between the patient's face and the cushion.

The superior-facing intermediate portions 3121 may be located around some or all of the inferior periphery of the patient's nose in use. For example, the superior-facing intermediate portions 3121 may be configured to lie just outside of a patient's nose at the base of the nose, e.g. proximate or in contact with the ala. The superior-facing intermediate portions 3121 of the seal-forming structure 3100 may comprise a pair of exterior walls facing towards partially medial and partially superior directions (e.g. having external or exterior surfaces facing in medial and superior directions) and in some examples also partially posteriorly. The seal-forming structure 3100 comprises a greater stiffness in the superior-facing intermediate portions in comparison to the central portions. In this example, the seal-forming structure 3100 comprises a greater wall thickness in the intermediate portions 3121 and 3125 than in the central portions 3111, 3115 and lip superior portion 3116.

In general, a greater stiffness in regions of the seal-forming structure 3100 compared to other regions of the seal-forming structure 3100 may be provided by a greater wall thickness, a stiffer material (e.g. higher durometer silicone or other material), a reinforcing structure such as a tie or a rib, an undercushion, portion or a chassis, or the like, across various examples of the present technology.

In the examples of Figs. 3-11-1 to 3-12-5, the nasal portion has central saddle portion 3112 configured to seal to anterior or inferior areas of the patient's pronasale. The intermediate portions are, in these examples, provided to lateral sides of the nasal portion 3230 of the seal-forming structure 3100 but not to the central saddle portion 3112.

The seal-forming structure 3100 may have a wall thickness of between 0.15-0.4mm in the superior-facing central portion 3111, anterior-facing central portion 3115 and/or lip superior portion 3116, for example between 0.2mm and 0.3mm, such as 0.25mm. The wall thickness in the superior-facing intermediate portions 3121 and anterior facing intermediate portions 3125 may be in the range of 0.5mm-1mm, such as between 0.6mm-0. 9mm, for example 0.75mm.

In some examples, the superior-facing intermediate portions 3121 of the seal-forming structure 3100 reinforce the superior-facing central portion 3111 of the seal-forming structure 3100. In further examples, the superior-facing intermediate portions 3121 provides a barrier to creases and prevents leak paths from forming in the seal-forming structure 3100. Additionally, the superior-facing intermediate portions 3121 may provide flexibility and support against sideward loading on the plenum chamber 3200 to help prevent breaking the seal under lateral forces.

As the superior-facing central portion 3111 has a thin wall thickness and is therefore very flexible, it may be prone to creasing in some conditions. The superior-facing intermediate portions 3121 are stiffer than the superior-facing central portion 3111 due to greater wall thickness and are therefore less prone to creasing. The nasal portion 3230 of the seal-forming structure 3100 may be particularly prone to creasing proximate the sides of the patient's nose. If a crease begins in the seal-forming surface and continues outside of the seal forming surface, the crease may create a leak path through which gas can leak through the crease from the interior of the plenum chamber 3200 to ambient, past the patient's face.

The superior-facing intermediate portion 3121, by virtue of its increased wall thickness, resists creasing. If a crease occurs in the superior-facing central portion 3111 of the seal-forming structure 3100, the superior-facing intermediate portions 3121 can limit the size of the crease to prevent it from continuing up the patient-facing sides of the nasal portion 3230 and past the seal forming surface (e.g. part the periphery of the patient's nose). Accordingly, the superior-facing intermediate portions 3121 act as a barrier to creases that closely follow the shape of the patient's nose, by being configured to lie at or proximate the edges of the patient's ala around the base of the nose. Furthermore, the intermediate portions 3121 or 3125 may assist in resisting or preventing creases in the thinner central portions 3111 or 3115. When the patient dons the patient interface 3000, the intermediate portions may stretch the thinner central portion onto the base of the nose (e.g. to the inferior periphery). The increased resistance to deformation that is provided by the intermediate portions proximate the patient's ala may resist or prevent creases from forming in the central portion by stretching the central portion onto the base of the nose.

The seal-forming structure 3100 may also comprise a lateral-facing posterior portion 3141 on lateral non-patient facing regions of the nasal portion 3230 of the seal-forming structure 3100. The lateral-facing posterior portions 3103 of the nasal portion 3230 may comprise a greater stiffness than the superior-facing intermediate portions 3121 and anterior-facing intermediate portions 3125. The lateral-facing posterior portions 3141 may comprise a greater wall thickness than the intermediate portions, central portions and or lip superior portion 3116.

The superior-facing intermediate portions 3121 are positioned to interfere with the sides of the patient's nose when the patient dons the patient interface 3000, yet are flexible enough that they can be deformed by the patient's nose to deform laterally. The seal-forming structure 3100 is biased at the superior-facing intermediate portions 3121 into contact with the patient's nasal ala. The patient's nose exerts forces outwardly on the sides of the seal-forming structure 3100 when the patient dons the patient interface 3000. In turn, the sides of the seal-forming structure 3100 in the nasal portion 3230 conform to the periphery of the patient's nose to form a stable and robust seal. The stiffness of the seal-forming structure 3100 in the superior-facing intermediate portions 3121 is advantageously large enough that the nasal portion 3230 can fit to and create a robust seal with a narrow nose but is not so stiff that it would be uncomfortable for a wider nose.

Additionally, the preload on the sides of the nose provided by the superior-facing intermediate portions 3121 provides decoupling between the patient's nose and the rest of the plenum chamber 3200 and patient interface 3000, tolerating some lateral movement of some portions of the plenum chamber 3200, e.g. the shell 3210, without disrupting the seal in use.

The anterior areas of the nasal portion 3230 are configured to be comfortable while able to form a stable seal to the patient's nose.

The anterior-facing central portion 3115 has in some examples a thickness of approximately 0.15-0.4mm, for example 0.2-0.3mm, and in the illustrated example comprises a wall thickness of approximately 0.25mm. The anterior-facing intermediate portions 3125 may comprise a wall thickness that is greater than a wall thickness in the anterior-facing central portion 3115, and may be between 0.5mm and 1mm, such as between 0.65mm and 0.85mm or, in the illustrated examples, approximately 0.75mm. In some examples the thickness of the seal-forming structure 3100 tapers between regions having different thicknesses. In other examples the thickness changes relatively abruptly, e.g. in steps. The anterior areas of the nasal portion 3230 is configured to be somewhat compliant and is therefore not as thick and stiff as other regions of the nasal portion 3230, such as the posterior corners 3131 of the nasal portion 3230 (which may be in the range of 1-1.5mm in thickness). The anterior areas of the nasal portion 3230 may generally be sufficiently thick that it is stiff enough to hold its general shape when donned by a patient.

Providing flexibility in the anterior portion of the nasal portion 3230 allows the seal-forming structure 3100 to deform somewhat when receiving a patient's nose, especially a longer nose. The patient's pronasale can be a particularly sensitive area and the flexibility of the anterior portion of the nasal portion 3230 may help reduce forces applied to the patient's nose from the seal-forming structure 3100. This flexibility is particularly advantageous at the location of the pronasale, and therefore the central portions are less stiff than the anterior-facing intermediate portions 3125. The superior-facing central portions 3111 and anterior-facing central portions 3115 may be around 0.25mm in thickness while the anterior-facing intermediate portions 3125 may have a wall thickness of about 0.75mm. The reduced thickness of the central portions reduces pressure on the pronasale while the thicker intermediate portions 3125 provide some support to the overall structure of the nasal portion 3230.

A further advantage of a flexible central region of the nasal portion 3230 is that it enables the sides of the nasal portion 3230 to be pulled inwardly (e.g. in a medial direction) when the patient dons the patient interface 3000 and the patient's nose exerts a downward force on the superior-facing central portion 3111 of the seal-forming structure. The inwards pull on the sides of the nasal portion 3230 towards the sides of the patient's nose may improve the seal since the seal-forming structure 3100 is pulled into and around the inferior periphery of the patient's nose.

Although the sides of the nasal portion 3230 are pulled inwards, the anterior portion, and in particular the anterior-facing intermediate portions 3125 on either side of the more flexible anterior-facing central portion 3115, retains enough structural rigidity to maintain the overall shape of the seal-forming structure 3100 and prevent creases from creating leak paths. In alternative examples, the anterior-facing central portion 3115 may be closer in thickness to the anterior-facing intermediate portions 3125 to provide further crease resistance at the central saddle portion 3112 of the nasal portion 3230.

It is advantageous that the nasal portion 3230 of the seal-forming structure 3100, and in particular the region between the nasal holes 3272 and the central saddle region 3112 of the nasal portion 3230, is relatively long in the anterior-posterior directions. This part of the nasal portion 3230 of the seal-forming structure 3100 is relatively unsupported in comparison to other regions. A long area in this part of the seal-forming structure 3100 allows ample room for the nasal portion 3230 of the seal-forming structure 3100 to deform, which is advantageous as it receives the nose. If the seal-forming structure 3100 has a stiffer structure in this region, excessive force could be exerted on the patient's pronasale, especially in longer noses. It is advantageous to avoid this excessive force for comfort, given the pronasale can be a particularly sensitive region.

The superior-facing central portion 3111 of the nasal portion 3230 of the seal-forming structure 3100 has a bridge portion 3113, connecting the anterior region of the superior-facing central portion 3111 with the lip superior portion 3116 of the nasal portion. The bridge portion 3113 therefore lies inferior to the patient's columella in use and partly defines the two nasal holes 3272 (one on either lateral side) through which air can be supplied to the patient.

The bridge portion 3113 is flexible and curved to be slack when the patient interface 3000 is not donned by the patient. The slack provided to the bridge portion 3113 allows the anterior part of the central portion 3113 (anterior of the bridge, which contacts the patient's pronasale in use) to move away from the lip superior portion 3116 when the patient dons the patient interface 3000.

Advantageously, this allows a nose with a longer length to still be comfortably accommodated by the seal-forming structure 3100. A longer and/or narrower nose could comfortably push the anterior part of the superior-facing central portion 3111 in an anterior direction. By allowing deformation of the nasal portion 3230 in this way, the forces exerted by the seal-forming structure 3100 back onto the patient's pronasale (which is generally quite sensitive) can be kept to a tolerable level. The bridge portion 3113 is therefore advantageous in enabling the seal-forming structure to accommodate a range of nose sizes while still be comfortable and able to achieve a good seal.

The bridge portion 3113 may be S-shaped so that it can straighten out to tolerate movement of the superior-facing central portion 3111 of the seal-forming structure 3100 away from the lip superior portion 3116. The bridge portion 3113 may be bowed, curved or folded. The bridge portion 3113 may have concertina or bellows. The bridge portion 3113 may comprise only one curve so that the bridge is C-shaped, or may comprise two bows so that it is S-shaped, to provide even more slack. The bridge portion 3113 may be in the form of a sling (e.g. a portion that hangs between its end). The bridge portion 3113 is longer and is comprised of more material than is necessary to bridge the gap between the anterior and posterior portions of the superior-facing central portion 3111 of the nasal portion 3230. The extra material allows for extension while the bridge portion 3113 straightens out, before becoming taut. The more extra material is provided, the more extension can be provided by the bridge portion 3133 before it becomes taut upon a force applied to the anterior part of the superior-facing central portion 3111). Since the bridge portion 3113 does not need to seal to the patient's columella in order to create a full seal to the patient's nose, a good seal can be made to a small nose even if the bridge remains somewhat slack.

The bridge portion 3113 may have a thickness of about 0.2mm-045mm, or 0.3-0.4 mm for example 0.35mm. The bridge portion 3113 may have a greater material thickness in than the surrounding superior-facing central portion 3111 of the seal-forming structure 3100 which helps resist or prevent the bridge portion 3113 from tearing. The bridge portion 3113 could alternatively be wider and thinner. In this example, a thicker and narrower bridge portion 3113 is provided so that the nasal holes 3272 are relatively large.

The bridge portion 3113 may serve other purposes as well. For example, it may maintain the integrity of the central portion 3111 in the thin zone. If there were no bridge portion 3111 and instead a single nasal hole, due to the relatively thin wall thickness of the central portion, whilst under pressure if the seal-forming structure is refitted (e.g. via pulling of the face and reseating) or if it receives aggressive dynamic loading, there is a chance that blowout may occur at the superior-facing central portion 3111. The bridge portion 3113 ties the superior-facing central portion 3111 to the lip superior portion 3116 to reduce the chance of blowout at the superior-facing central portion 3111.

Additionally, the bridge portion 3113 may help prevent the patient from setting up the patient interface 3000 incorrectly. If the bridge portion 3113 was not provided and there was instead a single nasal hole, a patient may inadvertently insert their nose into the nasal hole. Since in this example the seal-forming structure 3100 is configured to seal to an inferior periphery of the patient's nose, the seal-forming structure may not achieve a suitable seal if the patient inserts their nose into the nasal hole.

Notwithstanding the advantages of the bridge portion 3113 in some forms of the technology, in some alternative examples there is no bridge portion 3113 provided and there is only a single hole in the nasal portion 3230 of the seal-forming structure 3100. This would allow the superior-facing central portion 3111 of the nasal portion 3230 to move relative to the inferior portion, and would also make the seal-forming structure easier to clean. However, there may be an increased risk of blowout if the superior-facing central portion of the seal-forming structure 3100 is not tied to the lip superior portion 3116. Additionally, the patient could attempt to insert their nose into the single hole. Provided steps are taken to mitigate these risks (e.g. by providing the seal-forming structure with a thicker, smaller or tighter membrane around the hole), then a single hole (i.e. no bridge) may be provided in alternative examples.

While, in the areas of the seal-forming structure 3100 discussed above, it is advantageous for the seal-forming structure 3100 to have a low wall thickness to allow it to comfortably conform to complex geometry, in some regions of the seal-forming structure 3100 a relatively thicker wall thickness is advantageous in other forms of the technology.

For example, the nasal portion 3230 of the seal-forming structure 3100 comprises posterior corners 3131 configured to seal to the patient's face proximate the nasaolabial creases. These posterior corners 3131 have a greater wall thickness in comparison to the central portion of the nasal portion 3230 of the seal-forming structure 3100. The greater wall thickness provides significant structural rigidity to the seal-forming structure 3100 in these regions, which may provide a number of advantages.

The posterior corners 3131 may assist in supporting the seal-forming structure on the patient's face and therefore need to have sufficient structural rigidity so that they do not collapse and compromise the seal achieved by the central portion of the seal-forming structure 3100 (e.g. the superior-facing central portion 3111 and/or lip superior portion 3116). In alternative examples, the posterior corners 3131 are provided with an undercushion for reinforcement. In the illustrated examples in which a single wall seal-forming structure 3100 is provided, structural rigidity is provided by a sufficiently high wall thickness, which may be around 0.8-1.6mm thick, for example 1.1mm to 1.45mm, or 1.25mm in one example. The posterior corners 3131 are configured to lie on the patient's face in a region inferior to the ala of the patient's nose as well as inferior and laterally outwards of the patient's nose, for example between the nasolabial sulcus and the regions of the lip superior located inferior to the ala.

As shown in Figs. 3-5J, 3-10J, 3-11-1 and 3-12-1, there is an abrupt transition (e.g. an abrupt taper or step) between the higher wall thickness in the posterior corners 3131 and the low wall thickness in the lip superior portion 3116 of the seal-forming structure 3100 (i.e. the region that seals against the lip superior). The thick posterior corners 3131 may provide support to the seal-forming structure where support is required/advantageous while the lip superior portion 3116 lies against and seal to against the patient's lip superior and may be much more flexible to conform to the contours of the patient's face while keeping forces to a minimum. While facial geometry can vary widely between patients, the abrupt transitions may be located almost directly inferior to the nasal ala on the lip superior.

As shown in Figs. 3-11-1 and 3-12-1, superior to the location of the junction between the thicker of the posterior corners 3131 and the thinner wall thickness at the lip superior portion 3116 of the nasal portion 3230 of the seal-forming structure 3100 are lateral corner regions 3114 of the superior-facing central portion 3111 that extend in a lateral, posterior and superior direction on either side of the target seal forming region (e.g. they extend up the medially-facing sides of the seal-forming structure 3100). The seal-forming structure 3100 is configured so that the lateral corner regions 3114 of the central portion contact and seal against the ala. The ala can be significantly curved, and many patients may have very concave pockets or cavities where the ala meets the face. The pockets can result from ala that curve back towards a medial direction (e.g. towards the sagittal plane) between the widest part of the nose and the junction of the ala and the face. Providing a low stiffness (which in this example is achieved by a low wall thickness) at these lateral corner regions 3114 enables the seal-forming structure 3100 to deform to match the curvature of the ala. This flexibility and ability to conform may assist the seal-forming structure 3100 in filling the concavities which may exist at the inferior corners of the patient's nose.

The seal-forming structure 3100 may be configured so that each transition between the thick posterior corner 3131 and the thin lip superior portion 3116 may be located on the patient's face laterally of the ala but close to the ala. The regions of the seal-forming structure 3100 contacting the patient's face on either inferior lateral side of the nose may be part of the thick posterior corners 3131 to provide good support and stability to the seal-forming structure 3100. Additionally, the lateral corner regions 3114 of the superior-facing central portion 3111 may deform to receive the patient's ala. For example, the lateral corner regions 3114 may cup the patient's ala. They may deform to create shelves in the cushion on which the proximal portions of the patient's ala may be supported in use, enabling the seal-forming structure 3100 to conform well to the inferior periphery of the patient's nose in use, especially proximate the lip superior.

The boundary between the thick walls forming the posterior corners 3131 and the medially adjacent portions of the seal-forming structure 3100 may track upwardly, outwardly and then inwardly to follow the curvature of the patient's nose in a superior direction starting from under patient's ala. The medial boundaries of the walls forming the thick posterior corners 3131 may follow a path up the patient's face on either side of the nose following the curvature along either side of the nose. This may provide good support where needed (e.g. against the patient's face just below and on either side of the patient's nose) while enabling the thinner portions, such as the superior-facing central portion 3111 and the lip superior portion 3116 to conform and seal to the ala and underside of the patient's nose.

As shown in the side views such as Figs 3-11-5 and 3-12-5, the seal-forming structure 3100 is even thicker towards its anterior side closer to the shell 3210 (not shown in Figs, 3-11-5 or 3-12-5 but shown in Figs 3-1-3 and 3-6-3). The thicker regions of the seal-forming structure 3100 proximate the shell 3210 provide good support and structural rigidity to the seal-forming structure 3100. As discussed above, the seal-forming structure 3100 comprises lateral support portions 3131 in the form of thickened regions on the partially-anterior facing lateral sides of the nasal portion 3230 of the seal-forming structure 3100.

While thick areas proximate the shell 3210 are advantageous in providing structural rigidity, the nasal portion 3230 of the seal-forming structure 3100 still retains a degree of flexibility to enable the sides of the seal-forming structure 3100 to be pushed outwardly or pulled inwardly to accommodate noses of different widths.

For example, the non-patient facing sides or regions (e.g. anterior sides, at least partially anterior-facing sides) of the nasal portion 3230 of the seal-forming structure 3100 (particularly the non-patient contacting regions on either side of nasal portion 3230 of the seal-forming structure 3100) are thick enough to provide sufficient structural rigidity to the seal-forming structure 3100 but are thin enough so that when the seal-forming structure 3100 is donned by a patient with a long narrow nose, the downward forces exerted by the patient's nose on the superior-facing central region 3111 are able to pull the sides of the nasal portion 3230 inwardly somewhat to bring the patient-contacting surfaces of the seal-forming structure 3100 on either side of the patient's nose into good contact with the patient's nose. Similarly, if a patient with a wider nose dons the seal-forming structure 3100, the structure of the nasal portion 3230 of the seal-forming structure 3100 is sufficiently flexible that there are not excessive inwards forces on the sides of the patient's nose (which may occur if the seal-forming structure is too stiff to tolerate a wider nose). Of course, a number of different sizes for the seal-forming structure 3100 are also able to be provided to accommodate different ranges of nose widths.

As shown in Figs. 3-1-1 and 3-6-1, there is an oronasal transition 3275 at the periphery of the plenum chamber 3200 where the nasal portion 3230 and oral portion 3260 connect. The periphery of the seal-forming structure 3100 varies at this location between examples of the technology. As shown in Fig. 3-1-1 the oronasal transition 3275 is relatively sharp and there is relatively large positive curvature at the periphery of the seal-forming structure 3100 between the nasal portion 3230 and oral portion 3260. In contrast, as shown in Fig. 3-6-1, the oronasal transition 3275 is relatively gradual and there is relatively small positive curvature at the periphery of the seal-forming structure 3100 between the nasal portion 3230 and oral portion 3260. In both cases, the oronasal transition 3275 comprises a saddle region.

While the periphery of the seal-forming structure 3100 should preferably be sufficiently stiff that it can support the overall shape of the seal-forming structure 3100 and prevent large creases and buckling, the shape of the periphery may be varied more than the regions that are in contact with the patient's face and the nearby regions (i.e. the thin zones and the thicker zones which prevent creases from creating leak paths past the patient's face). In any case, the oronasal transition 3275 between the nasal portion and oral portions of the seal-forming structure 3100 are preferably relatively stiff (e.g. by being thick) in order to prevent creases or buckling from occurring and creating leaks between theses portions. Alternatively, the oronasal transition 3275 may be reinforced by any suitable means such as an undercushion, ribs, a portion of the shell or a frame or the like.

In some examples, the oral portion 3260 of the seal-forming structure 3100 comprises features to prevent creasing. The lateral periphery of the oral hole 3271 may be more susceptible to creasing or buckling than the top and bottom portions of the oral hole 3271, due to the downward forces on the nasal portion of the cushion and the wide oval shape of the oral opening.

In the examples shown in Figs. 3-11-1 to 3-12-5, the seal-forming structure 3100 comprises an oral hole peripheral portion 3117 at the periphery of the oral holes 3271, which is thinner than other portions of the seal-forming structure. Additionally, the seal-forming structure in those examples comprises posterior-facing lateral portions 3135 which are thicker than the oral hole peripheral portion 3117 and which resist creasing and buckling in the cushion which could lead to leak paths forming.

In some examples, such as the plenum chamber 3200 shown in Fig. 3-13, the seal-forming structure comprises lateral peripheral support portions 3136 at opposite lateral sides of the oral hole 3271. In this example, the posterior-facing lateral portions 3135 form the lateral peripheral support portions 3136. The posterior-facing lateral portion 3135 extend medially towards the most lateral edges of the oral hole 3271 to provide the lateral peripheral support portions 3136. The lateral peripheral support portions 3136 provide extra resistance to buckling.

### 4.3.1.3.3 Oral-region

In one form, the non-invasive patient interface 3000 comprises a seal-forming structure 3100 that forms a seal in use on an upper lip region (that is, the lip superior) of the patient's face. The seal forming structure may comprise a lip superior portion 3116 configured to form a seal to the lip superior of the patient.

In one form, the seal-forming structure includes a saddle-shaped region constructed to form a seal in use on an upper lip region of the patient's face.

In one form the non-invasive patient interface 3000 comprises a seal-forming structure that forms a seal in use around the patient's mouth at an oral portion 3260. The seal-forming structure 3100 may form a seal on a chin-region of the patient's face.

In one form, the seal-forming structure includes a saddle-shaped region constructed to form a seal in use on a chin-region of the patient's face.

The seal-forming structure 3100 as shown in Figs. 3-11-1 to 3-12-5 comprises a lip inferior portion 3118 which forms a seal against the chin region of the patient. The lip inferior portion 3118 of the seal-forming structure may seal against the lip inferior and supramenton of the patient. Additionally, in this example, the seal-forming structure 3100 comprises an oral hole peripheral portion 3117. The lip inferior portion 3118 may be connected to (e.g. contiguous with) the lip superior portion 3116 via the oral hole peripheral portion 3117. The seal-forming structure 3100 comprises a relatively low wall thickness at the oral hole peripheral portion 3117 and at the lip inferior portion 3118 of the seal-forming structure 3100 which lies against the chin region (in comparison to other regions). The low wall thickness in these locations assists in achieving an effective, comfortable seal. The seal-forming structure 3100 in these regions is able to readily conform to any complex geometry (e.g. the labiomandibular creases).

In this example, the oral portion 3260 comprises posterior-facing lateral portions 3135 on the patient-contacting side of the seal-forming structure 3100. As discussed above, immediately around the oral hole 3271 at the oral hole peripheral portion 3117 the wall thickness is low in comparison to other regions of the seal-forming structure 3100, however in this example there are posterior-facing lateral portions 3135 on either lateral side of the oral hole peripheral portion 3117 which are thicker than the oral hole peripheral portion 3117. These regions may have a wall thickness of around 1mm to 1.5mm, such as 1.15 mm to 1.35mm, for example around 1.25mm thick. The areas that these regions contact in use, i.e. the patient's cheeks, may generally not be as sensitive as other areas of the face and therefore patients may generally tolerate the seal-forming structure 3100 having a greater wall thickness in these areas. Additionally, the posterior-facing lateral portions 3135 of the oral portion 3260 curve away from contact with the patient's face which reduces the area of contact on the patient's face at these regions. In alternative examples the posterior-facing lateral portions 3135 of the oral portion 3260 may not be thicker and may instead be stiffened by another means, such as by reinforcing structures (e.g. ribs), a stiffer material, an undercushion or the like.

The posterior-facing lateral portions 3135 may provide resistance to creases that may form proximate the oral hole 3271, thereby preventing leak paths from being created. The posterior-facing lateral portions 3134 may provide a barrier to creases that form at the thinner oral hole peripheral portion 3117, limiting the extent of creases away from the oral hole 3271. This function of the posterior-facing lateral portions 3135 may be similar to the crease resistance function provided by the superior-facing intermediate portions 3121 of the nasal portion 3230 described above.

The lip inferior portion 3118 of the oral opening 3260 is approximately half the width of the oral opening 3260 and centered inferior to the oral hole 3271. As described above, the lip inferior portion 3118 is very thin. The transitions between the thinner lip inferior portion 3118 and the thicker posterior-facing lateral portions 3135 on either lateral side may be configured to lie at or proximate a patient's labiomandibular creases. The lip inferior portion 3118 is wider at the periphery of the oral hole 3271 than at the lower periphery of the seal-forming structure 3100. The width of the lip inferior portion 3118 therefore tapers down from the oral hole 3271.

Further from contact with the patient (e.g. closer to the shell 3210) than the posterior-facing lateral portions 3135 at the lateral periphery of the oral portion 3260 are lateral portions 3145 of the oral portion 3260. In these examples, the lateral portions 3145 are thicker than the posterior-facing lateral portions 3135 of the oral portions. In some examples, the lateral portions 3145 of the oral portion have a thickness in the range of 1.5-2.2mm, such as 1.7-2mm. Most, or all, of the lateral portions 3145 are unlikely to be in contact with the patient's face in use and accordingly, patient comfort is a less important design consideration for these regions and the wall thickness can be higher in these regions than in the patient-contacting regions. The higher wall thickness may provide structural rigidity to the overall shape of the oral portion 3260 of the seal-forming structure 3100. In some examples, the further away from the patient's face a particular region of the seal-forming structure 3100 is, the thicker that region is, unless there is a reason for providing flexibility to that region (e.g. to enable the sides of the nasal portion of the seal-forming structure 3100 to deform). The lateral portions 3145 define a lateral periphery of the seal-forming structure 3100 in the oral portion 3145.

On the anterior side of the seal-forming structure 3100, the wall thickness is generally higher in this example, with the exceptions of the anterior sides of the nasal portion and the central inferior region of the oral portion of the seal-forming structure 3100). In this example the seal-forming structure 3100 comprises anterior-facing lateral portions 3155. The lateral anterior facing portions 3155 in this example have a wall thickness greater than the lateral portions 3145 (and posterior-facing lateral portions 3135). The anterior-facing lateral portions may have a wall thickness in the range of 1.7-2.7mm, for example in the range of 2.0-2.5mm. These thicker areas provide substantial support and structural rigidity to the overall shape of the seal-forming structure 3100. The lateral anterior-facing portions 3155, in combination with other portions of the seal-forming structure that are provided with high wall thickness, such as the lateral portions 3145, hold the general shape of the seal-forming structure 3100 and may resist creases and/or buckling etc., while the thinner areas provided to the patient-facing side of the seal-forming structure 3100 deform against the patient's face under the forces exerted on the plenum chamber 3200 by the positioning and stabilising structure 3300.

Also on the anterior side of the seal-forming structure 3100 are anterior support portions 3161 of the oral portion 3260. The anterior support portions 3161 are even thicker zones of the seal-forming structure 3100 at the base of the nasal portion on the anterior side, where the nasal portion joins the oral portion, proximate the frame, and at the inferior lateral corners of the oral portion 3260. The wall thickness in these regions may be in the range of 2.3-3.3mm, for example 2.5-3mm. These regions may provide even further structural stiffness to the seal-forming structure. The anterior support portions 3165 may have a greater wall thickness in comparison to the anterior-facing lateral portions 3155. Generally, the cushion has a greater wall thickness further away from the seal forming region although immediately proximate the shell 3210, the thickness may be similar to the thickness of the anterior-facing lateral portions 3155, even though the anterior support portions 3165 are thicker. The silicone immediately adjacent the shell 3210 in some examples may require a lesser wall thickness since it may be reinforced by the peripheral edge of the shell 3210.

### 4.3.1.3.4 Forehead region

In one form, the seal-forming structure forms a seal in use on a forehead region of the patient's face. In such a form, the plenum chamber may cover the eyes in use.

### 4.3.1.3.5 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.1.3.6 Surface finish

In some examples, different regions of the seal-forming structure 3100 comprise different surface finishes.

Referring to Fig. 3-15, the plenum chamber 3200 comprises a seal-forming structure 3100 comprising a nasal portion 3230 and an oral portion 3260. The The seal-forming structure comprises a first surface finish in the nasal portion and a second surface finish in the oral portion different from the first surface finish. In the illustrated example the region 3101 has a first surface finish and the region 3103 has a second surface finish. The boundary between the first surface finish and the second surface finish is identified by the lines 3102, which may contact the patient's cheeks in use.

A coefficient of friction between the seal-forming structure 3100 and the patient's face is greater in the oral portion 3260 than in the nasal portion 3230. The first surface finish in region 3101 may be configured to provide the nasal portion with a smooth feel on the patient's face, which may be more comfortable. The second surface finish in region 3103 may be configured to provide the oral portion with grippy contact on the patient's face, which may enable a more robust seal. In some examples, the first surface finish at region 3101 may be a frosted surface finish. In some embodiments the second surface finish at region 3103 may be a polished surface finish. The nasal portion 3230 in this example comprises a lip superior portion 3116 having the first surface finish.

The polished surface finish may have a grippy, sticky feel to it so that there is higher friction acting against movement of the seal-forming structure 3100 against the patient's face. This is generally desired as it helps prevent movement of the plenum chamber 3200 when donned by a patient, thereby assisting in maintaining a seal. However, patients may consider the feeling of a polished surface finish to be less comfortable on their face than a smoother feeling low-friction surface finish. Patients may tolerate the feeling of the polished finish on their cheeks and below their mouth, but may not tolerate the feeling of the polished surface on and around their nose, given the nose is generally a more sensitive area. Therefore, a polished finish is provided around the oral portion 3260, while a frosted finish is provided to the nasal portion 3230 of the seal-forming structure 3100, in this embodiment. The frosted finish at the nasal portion 3230 may also assist the seal-forming structure 3100 to move against the nose as it conforms to the surfaces around the nose, which may also help in forming a seal.

Additionally, the added grip provided by the higher friction in the region of the second surface finish 3103 assists the oral portion 3260 to maintain a seal during movement of the patient's lower jaw. The jaw may have a tendency to move relative to the head (and drop, in particular). The added grip helps the seal maintain its position around the patient's mouth during movement of the jaw.

The boundary 3102 between the polished finish at 3103 and the frosted finish at 3101 is close to the boundary between the nasal portion 3230 and the oral portion 3260 of the seal-forming structure 3100, slightly closer to the oral portion 3260 than the nasal portion 3230. The boundary 3102 lies across the seal-forming structure 3100 roughly perpendicular to a path on the oral portion 3260 circumscribing the oral hole 3271. At the periphery of the oral hole 3271 the boundary 3102 may lie on the lip superior. At the periphery of the oral portion 3260 of the seal-forming structure 3100 the boundary may lie proximate the patient's zygomatic bone.

### 4.3.2 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 4.3.3 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 4.3.4 Decoupling structure(s)

In one form the patient interface 3000 includes at least one decoupling structure, for example, a swivel or a ball and socket.

### 4.3.5 Connection port

Connection port 3600 allows for connection to the air circuit 4170.

### 4.3.6 Forehead support

In one form, the patient interface 3000 includes a forehead support 3700.

### 4.3.7 Anti-asphyxia valve

In one form, the patient interface 3000 includes an anti-asphyxia valve.

### 4.3.8 Ports

In one form of the present technology, a patient interface 3000 includes one or more ports that allow access to the volume within the plenum chamber 3200. In one form this allows a clinician to supply supplemental oxygen. In one form, this allows for the direct measurement of a property of gases within the plenum chamber 3200, such as the pressure.

### 4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

### 4.5.1 Oxygen delivery

In one form of the present technology, supplemental oxygen 4180 is delivered to one or more points in the pneumatic path, such as upstream of the pneumatic block 4020, to the air circuit 4170 and/or to the patient interface 3000.

### 4.6 HUMIDIFIER

### 4.6.1 Humidifier overview

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.7 BREATHING WAVEFORMS

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot*, is about 4s. The person typically breathes at a rate of about 15 breaths per minute (BPM), with Ventilation *Vent* about 7.5 L/min. A typical duty cycle, the ratio of *Ti* to *Ttot*, is about 40%.

### 4.8 RESPIRATORY PRESSURE THERAPY MODES

Various respiratory pressure therapy modes may be implemented by the RPT device 4000.

### 4.9 GLOSSARY

For the purposes of the present technology disclosure, in certain forms of the present technology, one or more of the following definitions may apply. In other forms of the present technology, alternative definitions may apply.

### 4.9.1 General

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. `Flow rate' is sometimes shortened to simply 'flow' or 'airflow'.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Patient*: A person, whether or not they are suffering from a respiratory condition.

*Pressure:* Force per unit area. Pressure may be expressed in a range of units, including cmF₂O, g-f/cm² and hectopascal. 1 cmF₂O is equal to 1 g-f/cm² and is approximately 0.98 hectopascal. In this specification, unless otherwise stated, pressure is given in units of cmH₂O.

The pressure in the patient interface is given the symbol *Pm*, while the treatment pressure, which represents a target value to be achieved by the mask pressure *Pm* at the current instant of time, is given the symbol *Pt.*

*Respiratory Pressure Therapy (RPT)*: The application of a supply of air to an entrance to the airways at a treatment pressure that is typically positive with respect to atmosphere.

*Ventilator*: A mechanical device that provides pressure support to a patient to perform some or all of the work of breathing.

### 4.9.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

### 4.9.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component*: The ability of the structure or component to resist deformation in response to an applied load. The load may be a force or a moment, e.g. compression, tension, bending or torsion. The structure or component may offer different resistances in different directions.

*Floppy structure or component:* A structure or component that will change shape, e.g. bend, when caused to support its own weight, within a relatively short period of time such as 1 second.

*Rigid structure or component*: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.9.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, *Ti* to total breath time, *Ttot.*

*Effort (breathing)*: The work done by a spontaneously breathing person attempting to breathe.

*Expiratory portion of a breathing cycle:* The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate,* patient *airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of one inspiratory portion of a respiratory flow rate waveform and the start of the following inspiratory portion of the respiratory flow rate waveform.

*Typical recent ventilation*: The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation* (*Vent*): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.9.3 Anatomy

### 4.9.3.1 Anatomy of the face

*Ala:* the external outer wall or "wing" of each nostril (plural: alar)

*Alare:* The most lateral point on the nasal *ala.*

*Alar curvature (or alar crest) point*: The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework*: The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane*: A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage*: A generally triangular plate of cartilage. Its superior margin is attached to the nasal bone and frontal process of the maxilla, and its inferior margin is connected to the greater alar cartilage.

*Lip, lower (labrale inferius)*:

*Lip, upper (labrale superius)*:

*Greater alar cartilage*: A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils)*: Approximately ellipsoidal apertures forming the entrance to the nasal cavity. The singular form of nares is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle*: The angle between the columella and the upper lip, while intersecting subnasale.

*Otobasion inferior*: The lowest point of attachment of the auricle to the skin of the face.

*Otobasion superior*: The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal)*: The nasal ridge is the midline prominence of the nose, extending from the Sellion to the Pronasale.

*Sagittal plane*: A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal)*: The nasal septal cartilage forms part of the septum and divides the front part of the nasal cavity.

*Subalare*: The point at the lower margin of the alar base, where the alar base joins with the skin of the superior (upper) lip.

*Subnasal point*: Located on the soft tissue, the point at which the columella merges with the upper lip in the midsagittal plane.

*Supramenton*: The point of greatest concavity in the midline of the lower lip between labrale inferius and soft tissue pogonion

### 4.9.3.2 Anatomy of the skull

*Frontal bone*: The frontal bone includes a large vertical portion, the *squama frontalis*, corresponding to the region known as the forehead.

*Mandible*: The mandible forms the lower jaw. The mental protuberance is the bony protuberance of the jaw that forms the chin.

*Maxilla*: The maxilla forms the upper jaw and is located above the mandible and below the orbits. The *frontal process of the maxilla* projects upwards by the side of the nose, and forms part of its lateral boundary.

*Nasal bones*: The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone.* The occipital bone is situated at the back and lower part of the cranium. It includes an oval aperture, the *foramen magnum*, through which the cranial cavity communicates with the vertebral canal. The curved plate behind the foramen magnum is the *squama occipitalis.*

*Orbit*: The bony cavity in the skull to contain the eyeball.

*Parietal bones*: The parietal bones are the bones that, when joined together, form the roof and sides of the cranium.

*Temporal bones*: The temporal bones are situated on the bases and sides of the skull, and support that part of the face known as the temple.

*Zygomatic bones*: The face includes two zygomatic bones, located in the upper and lateral parts of the face and forming the prominence of the cheek.

### 4.9.3.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx*: The larynx, or voice box houses the vocal folds and connects the inferior part of the pharynx (hypopharynx) with the trachea.

*Lungs*: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity*: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.9.4 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame*: Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane*: Membrane will be taken to mean a typically thin element that has, preferably, substantially no resistance to bending, but has resistance to being stretched.

*Plenum chamber*: a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal*: May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell*: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener*: A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent*: (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.9.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.9.5.1 Curvature in one dimension

The curvature of a plane curve at *p* may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 4.9.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance `as the crow flies'.)

### 4.9.5.3 Space curves

*Space curves*: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.9.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 31, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 4.11 REFERENCE SIGNS LIST

- 1000: Patient
- 1100: Bed partner
- 3000: Patient interface
- 3100: Seal-forming structure
- 3111: Superior-facing central portion of the nasal portion
- 3112: Central saddle portion
- 3113: Bridge portion
- 3114: Lateral corner regions of the central portion
- 3115: Anterior-facing central portion of the nasal portion
- 3116: Lip superior portion
- 3117: Oral hole peripheral portion
- 3118: Lip inferior portion
- 3121: Superior-facing intermediate portion of nasal portion
- 3125: Anterior-facing intermediate portion of nasal portion
- 3131: Posterior corners of the nasal portion
- 3135: Posterior-facing lateral portions of the oral portion
- 3136: Lateral peripheral support portions of the oral portion
- 3141: Lateral-facing posterior portions of the nasal portion
- 3145: Lateral portions of the oral portion
- 3151: Lateral support portions of the nasal portion
- 3152: Inferior boundary of the lateral support portion
- 3153: Superior boundary of the lateral support portion
- 3155: Anterior-facing lateral portions of the oral portion
- 3165: Anterior support portions of the oral portion
- 3200: Plenum chamber
- 3210: Shell
- 3211: Superior edge of the shell
- 3215: Posteriorly protruding portions
- 3218: Rim
- 3230: Nasal portion
- 3240: Inlet port
- 3232: Superior periphery of the nasal portion
- 3260: Oral portion
- 3271: Oral hole
- 3272: Nasal hole
- 3275: Oronasal transition
- 3300: Positioning and stabilising structure
- 3350: Frame
- 3351: Snap fit hooks
- 3400: Vent system
- 3600: Connection port
- 3700: Forehead support
- 4000: RPT device
- 4170: Air circuit
- 4200: Electrical components
- 4300: Algorithm
- 5000: Humidifier

## Claims

1. A plenum chamber (3200) for a patient interface (3000), the plenum chamber (3200) pressurisable to a therapeutic pressure of at least 6 cmH2O above ambient air pressure, the plenum chamber (3200) having an oral portion (3260) and a nasal portion (3230), the plenum chamber (3200) comprising:
a seal-forming structure (3100) constructed and arranged to form a seal with a region of the patient's face surrounding an entrance to the patient's airways, said seal-forming structure (3100) having one or more holes therein such that the flow of air at said therapeutic pressure is delivered to an entrance to the patient's nares and to the patient's mouth via the one or more holes, the seal-forming structure (3100) constructed and arranged to maintain said therapeutic pressure in the plenum chamber (3200) throughout the patient's respiratory cycle in use;
a shell (3210) having one or more plenum chamber inlet ports (3240) sized and structured to receive a flow of air at the therapeutic pressure for breathing by a patient, the shell (3210) supporting the seal-forming structure (3100),
wherein the shell (3210) is provided to the oral portion (3260) of the plenum chamber (3200), and substantially the entire nasal portion (3230) is formed by the seal-forming structure (3100); and
wherein the seal-forming structure (3100) comprises lateral support portions (3151) located on laterally-spaced at least partially anterior-facing sides of the nasal portion (3230), each lateral support portion (3151) provided at an inferior region of a lateral side of the nasal portion (3230), the lateral support portions (3151) each having a higher resistance to deformation in use in comparison to an anterior-facing central portion (3115) of the seal-forming structure (3100) between the lateral support portions (3151),
wherein the seal-forming structure (3100) comprises lateral-facing posterior portions (3141) provided between respective intermediate portions (3121, 3125) and the lateral support portions (3151) on each side of the nasal portion (3230), the seal-forming structure (3100) having a greater thickness in the lateral-facing posterior portions (3141) than in the intermediate portions (3121, 3125).

2. The plenum chamber (3200) of claim 1, wherein the lateral support portions (3151) comprise portions of the seal-forming structure (3100) having a greater thickness in comparison to the anterior-facing central portion (3115) of the seal-forming structure (3100).

3. The plenum chamber (3200) of claim 1 or claim 2, wherein the lateral support portions (3151) each have a curved superior boundary (3153).

4. The plenum chamber (3200) of any one of claims 1-3, wherein the lateral support portions (3151) are substantially fin-shaped.

5. The plenum chamber (3200) of any one of claims 1-4, wherein the seal-forming structure (3100) comprises a central portion configured to form a seal to an inferior periphery of the patent's nose surrounding the patient's nares and to the patient's lip superior in use, the central portion having a lesser thickness than the lateral support portions (3100).

6. The plenum chamber (3200) of claim 5, wherein the seal-forming structure (3100) comprises intermediate portions provided between the central portion and the lateral support portions (3151), the seal-forming structure (3100) having a greater thickness in the intermediate portions than in the central portion.

7. The plenum chamber (3200) of claim 6, wherein the seal-forming structure (3100) comprises a lesser thickness in the intermediate portions than in the lateral support portions (3151).

8. The plenum chamber (3200) of any of claims 1 to 7, wherein at least part of each intermediate portion (3121, 3125) is provided superior and anterior to a respective lateral support portion (3151).

9. The plenum chamber (3200) of any of claims 1 to 8, wherein at least part of each lateral-facing posterior portion (3141) is provided superior and posterior to a respective lateral support portion (3151).

10. The plenum chamber (3200) of any one of claims 1 to 9, wherein the nasal portion (3230) of the plenum chamber (3200) comprises posterior corners (3131) configured to lie on the patients face proximate nasolabial creases on the patient's face.

11. The plenum chamber (3200) of any one of claims 1 to 10, wherein the shell (3210) comprises posteriorly protruding portions (3215) reinforcing the seal-forming structure (3100) at a base of the nasal portion (3230).

12. The plenum chamber (3200) of any one of claims 1 to 11, wherein the lateral support portions (3151) each have a substantially flat inferior boundary (3152).

13. The plenum chamber of claim 12, wherein the flat inferior boundary (3152) is provided adjacent a superior edge (3211) of the shell (3210).

14. A patient interface (3000) comprising:
a plenum chamber (3200) according to any one of claims 1 to 14;
a positioning and stabilising structure (3300) to provide a force to hold the seal-forming structure (3100) in a therapeutically effective position on the patient's head, the positioning and stabilising structure (3300) comprising a tie, the tie being constructed and arranged so that at least a portion overlies a region of the patient's head superior to an otobasion superior of the patient's head in use; and
a vent structure (3400) to allow a continuous flow of gases exhaled by the patient from an interior of the plenum chamber (3200) to ambient, said vent structure (3400) being sized and shaped to maintain the therapeutic pressure in the plenum chamber (3200) in use;
wherein the patient interface (3000) is configured to allow the patient to breathe from ambient through their mouth in the absence of a flow of pressurised air through the plenum chamber inlet port (3240).

## Patentansprüche

1. Plenumkammer (3200) für eine Patientenschnittstelle (3000), wobei die Plenumkammer (3200) auf einen Therapiedruck von mindestens 6 cmH₂O über Umgebungsluftdruck druckbeaufschlagbar ist, die Plenumkammer (3200) einen Oralabschnitt (3260) und einen Nasalabschnitt (3230) hat und die Plenumkammer (3200) aufweist:
eine dichtungsbildende Struktur (3100), die so aufgebaut und angeordnet ist, dass sie eine Dichtung an einer Region des Gesichts des Patienten bildet, die einen Eingang zu den Luftwegen des Patienten umgibt, wobei die dichtungsbildende Struktur (3100) ein oder mehrere Löcher darin hat, so dass die Luftströmung mit dem Therapiedruck zu einem Eingang der Nasenlöcher des Patienten und des Munds des Patienten über das eine oder die mehreren Löcher abgegeben wird, und die dichtungsbildende Struktur (3100) so aufgebaut und angeordnet ist, dass sie den Therapiedruck in der Plenumkammer (3200) über den gesamten Atemzyklus des Patienten im Gebrauch aufrecht erhält;
eine Schale (3210) mit einem oder mehreren Plenumkammer-Einlassanschlüssen (3240), die so bemessen und strukturiert sind, dass sie eine Luftströmung mit dem Therapiedruck zur Atmung durch einen Patienten aufnehmen, wobei die Schale (3210) die dichtungsbildende Struktur (3100) stützt,
wobei die Schale (3210) am Oralabschnitt (3260) der Plenumkammer (3200) vorgesehen ist und im Wesentlichen der gesamte Nasalabschnitt (3230) durch die dichtungsbildende Struktur (3100) gebildet ist; und
wobei die dichtungsbildende Struktur (3100) Lateralstützabschnitte (3151) aufweist, die auf lateral beabstandeten, mindestens teilweise anterior weisenden Seiten des Nasalabschnitts (3230) liegen, wobei jeder Lateralstützabschnitt (3151) an einer inferior gelegenen Region einer Lateralseite des Nasalabschnitts (3230) vorgesehen ist und die Lateralstützabschnitte (3151) jeweils eine höhere Verformungsbeständigkeit im Gebrauch verglichen mit einem anterior weisenden Mittelabschnitt (3115) der dichtungsbildenden Struktur (3100) zwischen den Lateralstützabschnitten (3151) haben,
wobei die dichtungsbildende Struktur (3100) lateral weisende, posterior gelegene Abschnitte (3141) aufweist, die zwischen jeweiligen Zwischenabschnitten (3121, 3125) und den Lateralstützabschnitten (3151) auf jeder Seite des Nasalabschnitts (3230) vorgesehen sind, wobei die dichtungsbildende Struktur (3100) eine größere Dicke in den lateral weisenden, posterior gelegenen Abschnitten (3141) als in den Zwischenabschnitten (3121, 3125) hat.

2. Plenumkammer (3200) nach Anspruch 1, wobei die Lateralstützabschnitte (3151) Abschnitte der dichtungsbildenden Struktur (3100) aufweisen, die eine größere Dicke verglichen mit dem anterior weisenden Mittelabschnitt (3115) der dichtungsbildenden Struktur (3100) haben.

3. Plenumkammer (3200) nach Anspruch 1 oder Anspruch 2, wobei die Lateralstützabschnitte (3151) jeweils eine gekrümmte superior gelegene Grenze (3153) haben.

4. Plenumkammer (3200) nach einem der Ansprüche 1 bis 3, wobei die Lateralstützabschnitte (3151) im Wesentlichen rippenförmig sind.

5. Plenumkammer (3200) nach einem der Ansprüche 1 bis 4, wobei die dichtungsbildende Struktur (3100) einen Mittelabschnitt aufweist, der so konfiguriert ist, dass er eine Dichtung an einer inferior gelegenen Peripherie der Nase des Patienten, die die Nasenlöcher des Patienten umgibt, und an der Oberlippe des Patienten gelegen im Gebrauch bildet, wobei der Mittelabschnitt eine kleinere Dicke als die Lateralstützabschnitte (3100) hat.

6. Plenumkammer (3200) nach Anspruch 5, wobei die dichtungsbildende Struktur (3100) Zwischenabschnitte aufweist, die zwischen dem Mittelabschnitt und den Lateralstützabschnitten (3151) vorgesehen sind, wobei die dichtungsbildende Struktur (3100) eine größere Dicke in den Zwischenabschnitten als im Mittelabschnitt hat.

7. Plenumkammer (3200) nach Anspruch 6, wobei die dichtungsbildende Struktur (3100) eine kleinere Dicke in den Zwischenabschnitten als in den Lateralstützabschnitten (3151) aufweist.

8. Plenumkammer (3200) nach einem der Ansprüche 1 bis 7, wobei mindestens ein Teil jedes Zwischenabschnitts (3121, 3125) superior und anterior zu einem jeweiligen Lateralstützabschnitt (3151) gelegen vorgesehen ist.

9. Plenumkammer (3200) nach einem der Ansprüche 1 bis 8, wobei mindestens ein Teil jedes lateral weisenden, posterior gelegenen Abschnitts (3141) superior und posterior zu einem jeweiligen Lateralstützabschnitt (3151) gelegen vorgesehen ist.

10. Plenumkammer (3200) nach einem der Ansprüche 1 bis 9, wobei der Nasalabschnitt (3230) der Plenumkammer (3200) posterior gelegene Ecken (3131) aufweist, die so konfiguriert sind, dass sie auf dem Gesicht des Patienten in der Nähe von Nasolabialfalten auf dem Gesicht des Patienten liegen.

11. Plenumkammer (3200) nach einem der Ansprüche 1 bis 10, wobei die Schale (3210) nach posterior vorstehende Abschnitte (3215) aufweist, die die dichtungsbildende Struktur (3100) an einer Basis des Nasalabschnitts (3230) verstärken.

12. Plenumkammer (3200) nach einem der Ansprüche 1 bis 11, wobei die Lateralstützabschnitte (3151) jeweils eine im Wesentlichen flache, inferior gelegene Grenze (3152) haben.

13. Plenumkammer nach Anspruch 12, wobei die flache, inferior gelegene Grenze (3152) benachbart zu einer superior gelegenen Kante (3211) der Schale (3210) vorgesehen ist.

14. Patientenschnittstelle (3000), die aufweist:
eine Plenumkammer (3200) nach einem der Ansprüche 1 bis 14;
eine Positionier- und Stabilisierstruktur (3300), um eine Kraft bereitzustellen, um die dichtungsbildende Struktur (3100) in einer therapeutisch wirksamen Position auf dem Kopf des Patienten zu halten, wobei die Positionier- und Stabilisierstruktur (3300) ein Verbindungselement aufweist und das Verbindungselement so aufgebaut und angeordnet ist, dass mindestens ein Abschnitt über einer Region des Patientenkopfs superior zu einem Otobasion superior des Patientenkopfs im Gebrauch liegt; und
eine Entlüftungsstruktur (3400), um eine kontinuierliche Strömung von Gasen, die durch den Patienten ausgeatmet werden, aus einem Innenraum der Plenumkammer (3200) in die Umgebung zu ermöglichen, wobei die Entlüftungsstruktur (3400) so bemessen und geformt ist, dass sie den Therapiedruck in der Plenumkammer (3200) im Gebrauch aufrechterhält;
wobei die Patientenschnittstelle (3000) so konfiguriert ist, dass sie dem Patienten ermöglicht, bei fehlender Druckluftströmung durch den Plenumkammer-Einlassanschluss (3240) aus der Umgebung durch den Mund zu atmen.

## Revendications

1. Chambre de détente (3200) pour une interface patient (3000), la chambre de détente (3200) pouvant être mise sous pression à une pression thérapeutique d'au moins 6 cm H₂O au-dessus d'une pression d'air ambiante, la chambre de détente (3200) ayant une partie buccale (3260) et une partie nasale (3230), la chambre de détente (3200) comprenant :
une structure de formation de joint (3100) conçue et agencée pour former un j oint avec une région du visage du patient entourant une entrée dans les voies respiratoires du patient, ladite structure de formation de joint (3100) ayant un ou plusieurs trous en son sein de sorte que le flux d'air à ladite pression thérapeutique soit distribué à une entrée dans les narines du patient et dans la bouche du patient via le(s) trou(s), la structure de formation de joint (3100) étant conçue et agencée pour maintenir ladite pression thérapeutique dans la chambre de détente (3200) pendant tout le cycle respiratoire du patient lors de l'utilisation ;
une coque (3210) ayant un ou plusieurs orifices d'admission de chambre de détente (3240) dimensionnés et structurés pour recevoir un flux d'air à la pression thérapeutique destiné à être respiré par un patient, la coque (3210) supportant la structure de formation de joint (3100),
dans laquelle la coque (3210) est fournie à la partie buccale (3260) de la chambre de détente (3200), et sensiblement toute la partie nasale (3230) est formée par la structure de formation de joint (3100) ; et
dans laquelle la structure de formation de joint (3100) comprend des parties de support latérales (3151) situées sur des côtés au moins partiellement à orientation antérieure latéralement espacés de la partie nasale (3230), chaque partie de support latérale (3151) étant disposée au niveau d'une région inférieure d'un côté latéral de la partie nasale (3230), les parties de support latérales (3151) ayant chacune une résistance supérieure à la déformation lors de l'utilisation en comparaison avec une partie centrale à orientation antérieure (3115) de la structure de formation de joint (3100) entre les parties de support latérales (3151),
dans laquelle la structure de formation de joint (3100) comprend des parties postérieures à orientation latérale (3141) disposées entre des parties intermédiaires respectives (3121, 3125) et les parties de support latérales (3151) de chaque côté de la partie nasale (3230), la structure de formation de joint (3100) ayant une épaisseur supérieure dans les parties postérieures à orientation latérale (3141) à celle dans les parties intermédiaires (3121, 3125).

2. Chambre de détente (3200) selon la revendication 1, dans laquelle les parties de support latérales (3151) comprennent des parties de la structure de formation de joint (3100) ayant une épaisseur supérieure en comparaison avec la partie centrale à orientation antérieure (3115) de la structure de formation de joint (3100).

3. Chambre de détente (3200) selon la revendication 1 ou la revendication 2, dans laquelle les parties de support latérales (3151) ont chacune une frontière supérieure incurvée (3153).

4. Chambre de détente (3200) selon l'une quelconque des revendications 1-3, dans laquelle les parties de support latérales (3151) sont sensiblement en forme d'ailettes.

5. Chambre de détente (3200) selon l'une quelconque des revendications 1-4, dans laquelle la structure de formation de joint (3100) comprend une partie centrale configurée pour former un joint avec une périphérie inférieure du nez du patient entourant les narines du patient et avec la lèvre supérieure du patient lors de l'utilisation, la partie centrale ayant une épaisseur inférieure à celle des parties de support latérales (3100).

6. Chambre de détente (3200) selon la revendication 5, dans laquelle la structure de formation de joint (3100) comprend des parties intermédiaires disposées entre la partie centrale et les parties de support latérales (3151), la structure de formation de joint (3100) ayant une épaisseur supérieure dans les parties intermédiaires à celle dans la partie centrale.

7. Chambre de détente (3200) selon la revendication 6, dans laquelle la structure de formation de joint (3100) comprend une épaisseur inférieure dans les parties intermédiaires à celle dans les parties de support latérales (3151).

8. Chambre de détente (3200) selon l'une quelconque des revendications 1 à 7, dans laquelle au moins une partie de chaque partie intermédiaire (3121, 3125) est disposée de façon supérieure et antérieure à une partie de support latérale respective (3151).

9. Chambre de détente (3200) selon l'une quelconque des revendications 1 à 8, dans laquelle au moins une partie de chaque partie postérieure à orientation latérale (3141) est disposée de façon supérieure et postérieure à une partie de support latérale respective (3151).

10. Chambre de détente (3200) selon l'une quelconque des revendications 1 à 9, dans laquelle la partie nasale (3230) de la chambre de détente (3200) comprend des coins postérieurs (3131) configurés pour reposer sur le visage du patient à proximité de plis naso-labiaux sur le visage du patient.

11. Chambre de détente (3200) selon l'une quelconque des revendications 1 à 10, dans laquelle la coque (3210) comprend des parties faisant saillie de façon postérieure (3215) renforçant la structure de formation de joint (3100) au niveau d'une base de la partie nasale (3230).

12. Chambre de détente (3200) selon l'une quelconque des revendications 1 à 11, dans laquelle les parties de support latérales (3151) ont chacune une frontière inférieure sensiblement plate (3152).

13. Chambre de détente selon la revendication 12, dans laquelle la frontière inférieure plate (3152) est disposée adjacente à un bord supérieur (3211) de la coque (3210).

14. Interface patient (3000) comprenant :
une chambre de détente (3200) selon l'une quelconque des revendications 1 à 14 ;
une structure de positionnement et de stabilisation (3300) pour fournir une force pour maintenir la structure de formation de joint (3100) dans une position thérapeutiquement efficace sur la tête du patient, la structure de positionnement et de stabilisation (3300) comprenant une attache, l'attache étant conçue et agencée de sorte qu'au moins une partie recouvre une région de la tête du patient supérieure à une otobasion supérieure de la tête du patient lors de l'utilisation ; et
une structure de ventilation (3400) pour permettre un flux continu de gaz exhalés par le patient depuis un intérieur de la chambre de détente (3200) dans l'atmosphère ambiante, ladite structure de ventilation (3400) étant dimensionnée et formée pour maintenir la pression thérapeutique dans la chambre de détente (3200) lors de l'utilisation ;
dans laquelle l'interface patient (3000) est configurée pour permettre au patient de respirer depuis l'atmosphère ambiante par le biais de sa bouche en l'absence d'un flux d'air sous pression à travers le port d'admission de chambre de détente (3240).
